(19) **Europäisches Patentamt**
**European Patent Office**
**Office européen des brevets**

(11) **EP 3 388 073 A1**

(12) **EUROPEAN PATENT APPLICATION**

(43) Date of publication:
**17.10.2018 Bulletin 2018/42**

(51) Int Cl.:
*A61K 39/00* (2006.01)          *A61K 39/395* (2006.01)
*G01N 33/50* (2006.01)          *G01N 33/68* (2006.01)

(21) Application number: **17166362.8**

(22) Date of filing: **12.04.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **polybliocept GmbH**
**63755 Alzenau (DE)**

(72) Inventors:
• **Geyer, Jakob**
  **28237 Bremen (DE)**
• **Mäurer, Markus**
  **184 44 Äkersberga (SE)**
• **Dodoo, Ernest**
  **112 34 Stockholm (SE)**

(74) Representative: **Patent- und Rechtsanwälte**
**Ullrich & Naumann**
**PartG mbB**
**Schneidmühlstrasse 21**
**69115 Heidelberg (DE)**

(54) **TARGETING MESOTHELIN IN BRAIN CANCER**

(57)    The present invention relates to a mesothelin binding protein for use in the treatment of brain cancer in a patient, wherein said mesothelin binding protein comprises an epitope binding domain which specifically binds to an epitope containing amino acid sequence that is similar or identical to a section of the amino acid sequence of mammalian mesothelin precursor, in particular human mesothelin precursor (SEQ ID NO: 1). The invention further relates to a CAR T-cell, and expanded T-cell or a cancer vaccine comprising a mesothelin binding protein for use in brain cancer therapy. Finally, the invention relates to a method for diagnosing a patient suspected of a brain tumor.

Fig. 5

**EP 3 388 073 A1**

**Description**

**FIELD OF THE INVENTION**

The present invention relates to mesothelin binding proteins, including anti-mesothelin antibodies, chimeric antibody receptors and expanded T-cells for the treatment of brain cancer, in particular glioblastoma multiforme (GBM). The invention further relates to a novel method for diagnosis of brain cancer based on mesothelin.

**BACKGROUND OF THE INVENTION**

In 2014, the World Health Organisation estimated that circa 2% of all human cancers occur in the central nervous system (CNS) [1]. Although significantly less frequent than other solid tumours i.e. lung cancer, melanoma, pancreatic cancer etc., CNS cancers are immensely morbid and have a very poor prognosis in patients. Commonly observed forms of primary (as well as malignant) glioma include pilocytic/diffuse astrocytomas (WHO grades I-II), oligodendroglioma (OD, WHO grade II-III), low-grade/anaplastic oligoastrocytoma (OA, WHO grades II-III) and glioblastoma multiforme (GBM, WHO grade IV) [1-3].

The most common and aggressive clinical manifestation of glioma is GBM, which has a 5-year survival rate of less than 3% [1], compared to the other primary gliomas listed here, which have a 5-year survival rate of at least 50% [1]. Patients diagnosed with GBM are treated with the alkylating agent temozolomide as a first-line chemotherapeutic, which is nevertheless rather toxic and is associated with suppression of bone marrow output [5].

The standard treatment for GBM comprises radiotherapy in conjunction with adjuvant temozolomide, an oral alkylating agent which has proven anti-tumour effects [4]. Also temozolomide is rather toxic and is associated with suppression of bone marrow output [5].

Qualifying patients undergo surgical resection of the tumour, although the prognosis thereafter is still rather meagre (max 17 months survival post-surgery) since most individuals experience recurrence [6]. Salvage therapy with Gamma Knife Surgery (GKS) for selective removal of the tumour mass (as opposed to whole-brain radiotherapy) has shown to modestly improve the median survival of patients with recurrent GBM, especially when administered adjunctively to standard chemotherapy and immunotherapy (anti-vascular endothelial growth factor (VEGF) monoclonal antibody, bevacizumab (Avastin®) [6].

Nevertheless, the tumour volume, rate of diffusion and response to adjuvant therapy collectively affect the GKS efficacy and treatment outcome for patients. Cell-based therapies are being studied for reduced toxicity, improved therapeutic performance and extended life span of patients with malignant gliomas [7].

Accordingly, there remains a need in the art for treatment strategies for brain cancer.

**SUMMARY OF THE INVENTION**

The present invention is *interalia* based on the finding that mesothelin is expressed strongly in tumor cells of brain cancer patients and on the identification of distinct immune recognition hotspots in full-length mesothelin of brain tumor cells that are particularly well targeted. Together with the finding that brain cancer patient-derived peripheral blood (PB) lymphocytes contain T-cells specifically targeting mesothelin epitopes corresponding to the hot spots as shown by IFN-γ responses, this leads to the novel use of mesothelin binding proteins for the treatment of brain cancer.

Thus according to a first aspect, the present invention relates to a mesothelin binding protein for use in the treatment of brain cancer in a patient, wherein said mesothelin binding protein comprises an epitope binding domain, which specifically binds to an epitope containing amino acid sequence that is similar or identical to a section of the amino acid sequence of mammalian mesothelin precursor, in particular human mesothelin precursor (SEQ ID NO: 1).

Examples of such a mesothelin binding protein are an anti-mesothelin antibody or a fusion protein containing such antibody, chimeric antibody receptor (CAR), or a T-cell receptor (TCR).

Thus according to a second aspect, the invention provides an expanded T-cell for use in the treatment of brain cancer in a patient, wherein said T-cell comprises a TCR according to the first aspect.

Based on the findings of the inventors, also mesothelin itself or fragments thereof can also be used in the treatment of brain cancer patients, e.g. as cancer vaccine. Therefore, according to a third aspect, the invention relates to a vaccine protein for use as cancer vaccine in the treatment of a brain cancer in a patient, comprising an amino acid sequence that is similar or identical to the amino acid sequence of the human mesothelin precursor or a fragment thereof.

Moreover, a correlation between the level of IFN-γ production of lymphocytes from PB of a brain cancer patient and the severity, i.e. the grade of brain cancer was identified. The grading based on the IFN-γ production may additionally be combined with the measurement of the mesothelin concentration in peripheral blood of the patient to further improve the accuracy of diagnosis of the tumor grade. Therefore, according to a fourth aspect, the invention provides a method for diagnosing a patient suspected of a brain tumor, comprising the steps of

a. Providing an unstimulated blood sample from the patient;

b. Culturing the cells in the blood sample in the presence of a cytokine cocktail comprising at least two cytokines selected from interleukin 2 (IL-2), interleukin 15 (IL-15) and interleukin 21 (IL-21) and a mesothelin peptide comprising an amino acid sequence that is similar or identical to the amino acid sequence of the human mesothelin precursor or a fragment thereof

c. Measuring the IFN-γ concentration in the culture;

d. Comparing the concentration of IFN-γ to an IFN-γ threshold;

wherein a concentration of IFN-γ above the IFN-γ threshold is indicative for a grade III or IV glioblastoma.

**FIGURES**

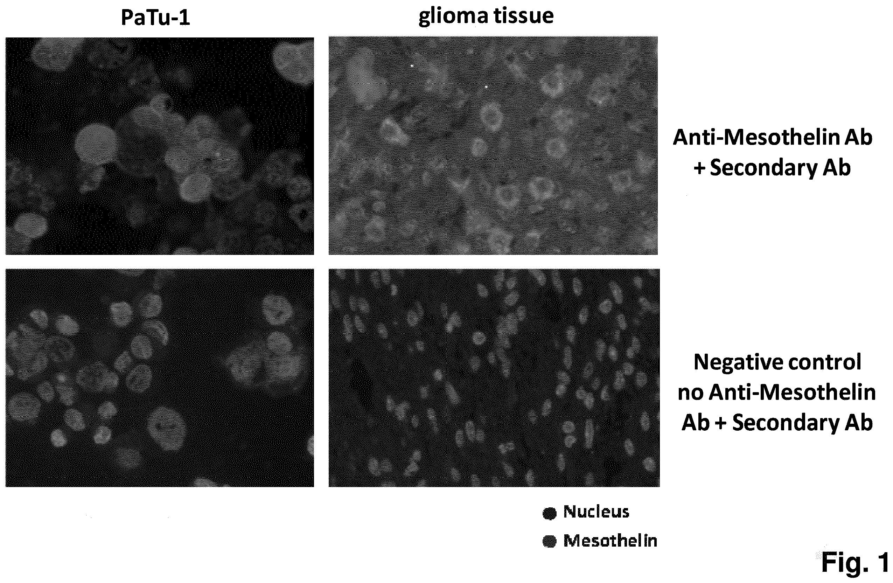

**Fig. 1** shows an immunohistological analysis of mesothelin overexpression in GBM tissue. Paraffin-embedded tissue sections were stained with an anti-mesothelin primary antibody. Detection was by an Alexa Fluor 488-conjugated secondary antibody. Magnification: 40x; green: mesothelin; blue: DAPI stained Nuclei.

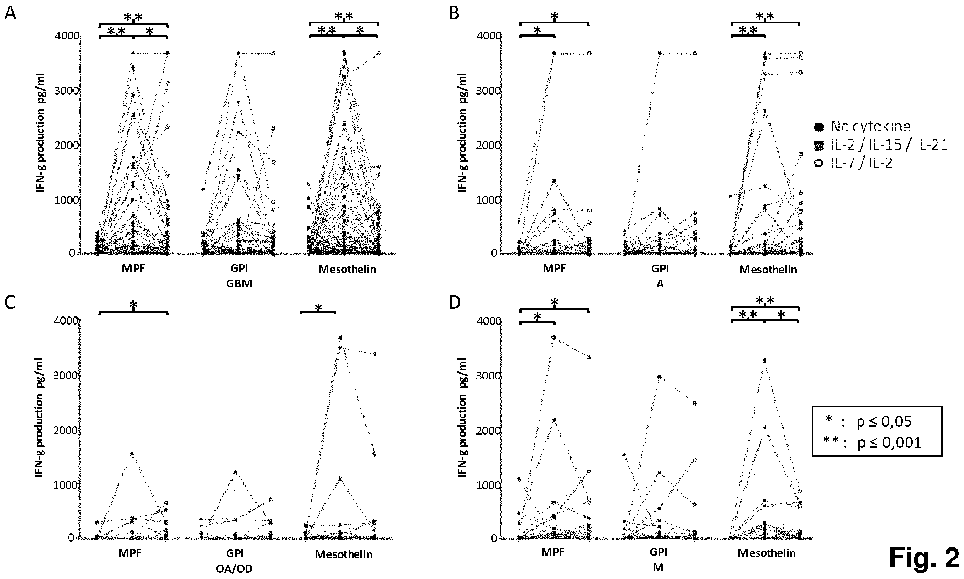

**Fig. 2** shows whole-blood IFN-γ responses of glioma patients to full-length mesothelin, MPF and GPI-anchored component with or without cytokine conditioning. Whole-blood obtained from patients with glioma (GBM, A; astrocytoma, B; OA/OD, C; metastasis, D) were cultured with mesothelin or its derivatives in the absence of cytokine conditioning, with IL-2/IL-7, or IL-2/IL-15/IL-21 conditioning over seven days. Supernatants were then harvested for IFN-γ detection by ELISA. Shown are dot plots representing responses of individual patients. Mann Whitney test of medians was performed to gauge statistical significance. *p<0.05; **p<0.001.

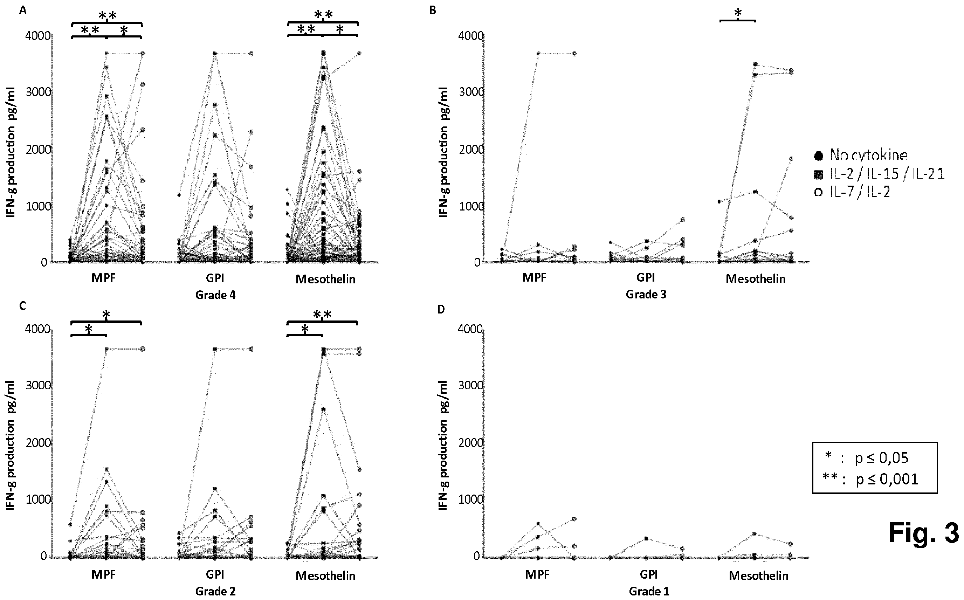

**Fig. 3** shows whole-blood IFN-γ responses to full-length mesothelin, MPF and GPI-anchored component with or without cytokine conditioning based on glioma grading. The data presented in Experiment 2 were reanalyzed based on WHO tumor grading. Shown are dot plots representing responses of individual patients. Mann Whitney test of medians was performed to gauge statistical significance. *p<0.05; **p<0.001.

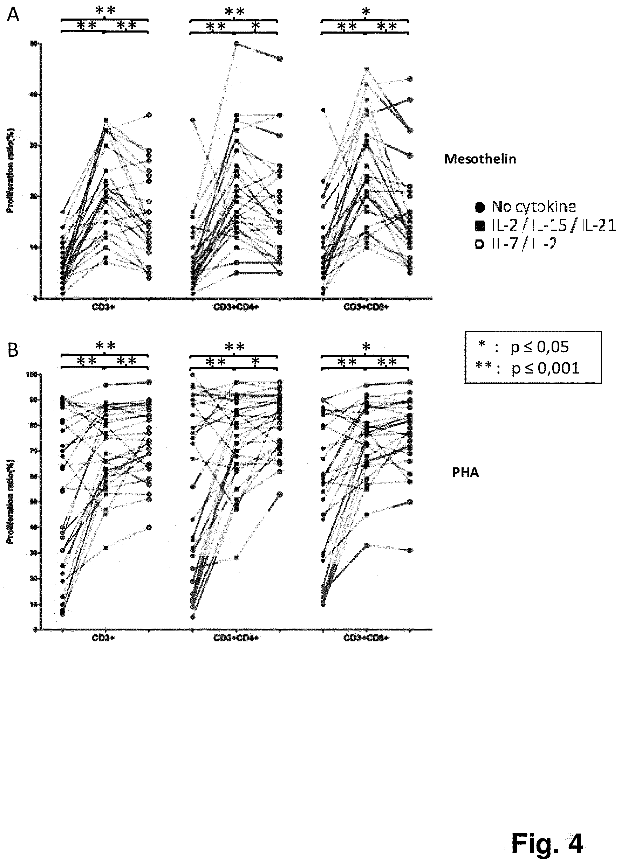

**Fig. 4** shows GBM peripheral blood T-cell proliferation in response to mesothelin stimulation with or without cytokine conditioning. Immune cells remaining in the culture plate at the end of the Example 2 were purified, processed and stained with CD3, CD4 and CD8 antibodies for flow cytometric analysis Shown are dot plots representing responses of individual patients. Mann Whitney test of medians was performed to gauge statistical significance. *p<0.05; **p<0.001.

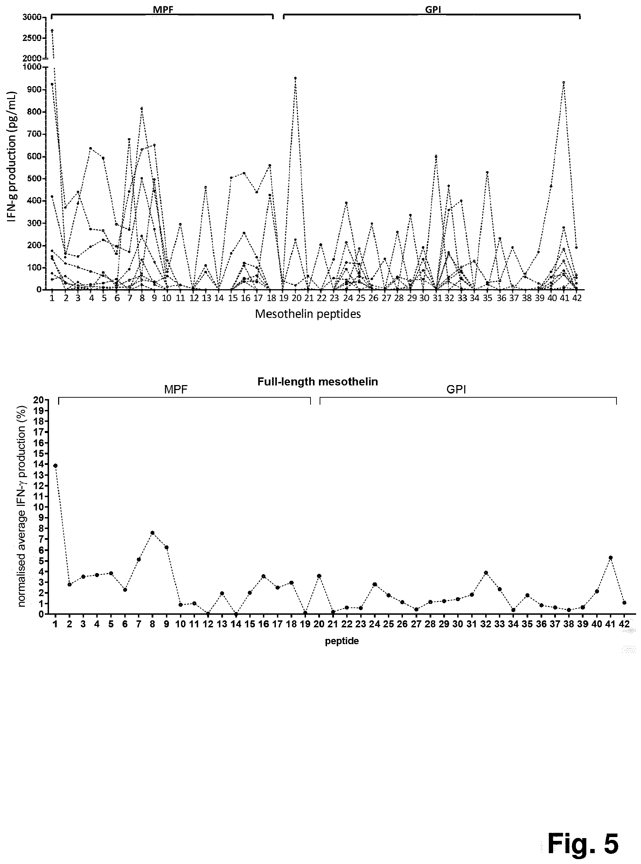

**Fig. 5** shows whole-blood IFN-γ responses to MPF and GPI-anchored component peptides. 42 non-overlapping 15-mer peptides spanning the entire mesothelin molecules were exposed to peripheral blood of GBM patients over a seven-day period. Supernatants were then harvested for IFN-γ detection by ELISA. Absolute IFN-γ concentrations (pg/ml) produced by each patient for a single peptide as well as the average value per peptide (total IFN-γ/no. of patients) normalised to the sum of IFN-γ production for the entire peptide pool in percentage are shown.

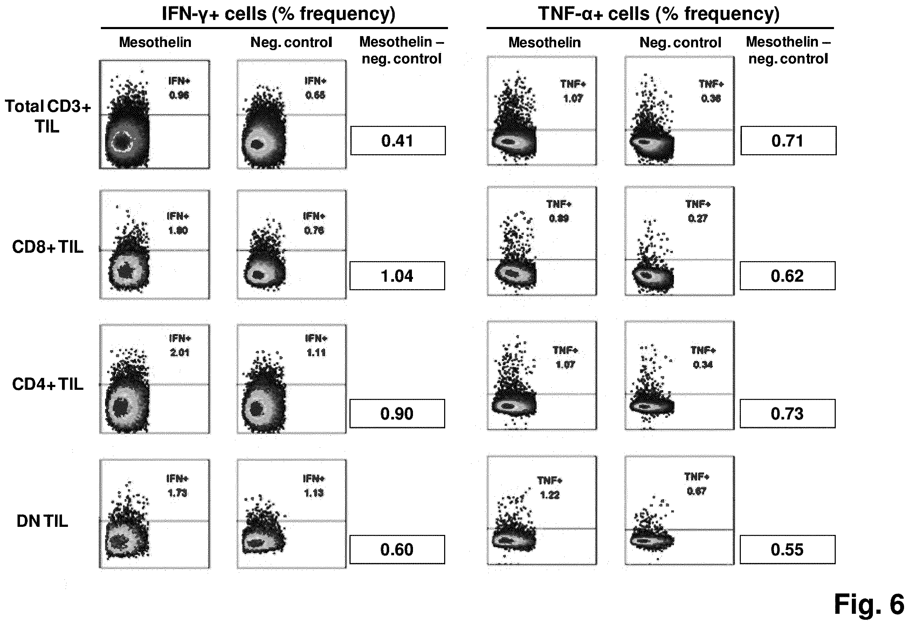

**Fig. 6** shows the recognition of mesothelin peptides by GBM TIL. TIL isolated from GBM tumour tissue and cultured *in vitro* with IL-2/IL-15/IL-21 were exposed to mesothelin peptides over a 6-hour period. Cells were then stained with CD3, CD4, CD8, IFN-γ and TNF-α to visualise intracellular cytokine production by mesothelin-specific T-cells. Shown are frequencies (%) of responding cells based on total TIL of one representative patient. Cell frequency of more than 0.2% is a considered a legitimate response.

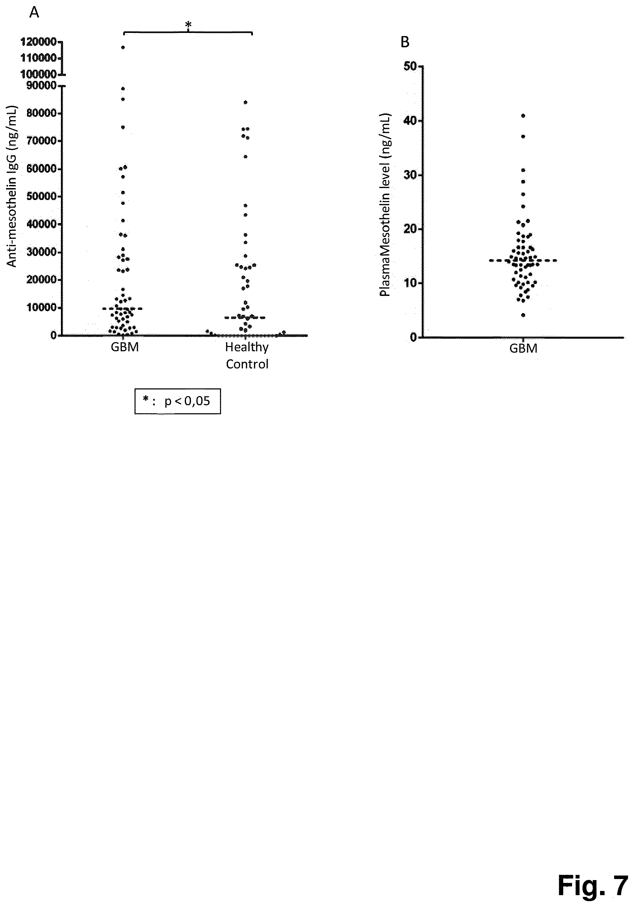

**Fig. 7** shows the detection of mesothelin-specific circulating IgG as well as shed mesothelin protein in plasma of GBM patients. An indirect ELISA method was used for quantifying mesothelin-specific IgG titres in plasma, while a commercially available ELISA kit was used for measuring mesothelin protein. IgG titres and mesothelin levels are expressed as ng/ml of plasma. Mann Whitney test of medians was performed to gauge statistical significance. *p<0.05; **p<0.001.

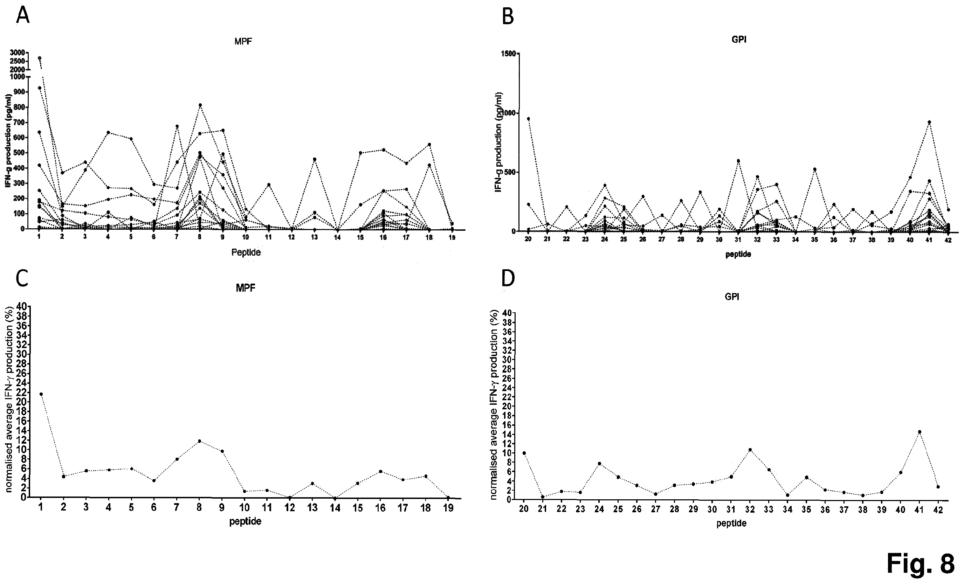

**Fig. 8** shows whole-blood IFN-γ responses specifically to (A and C) MPF and (B and D) GPI-anchored component peptides of mesothelin.

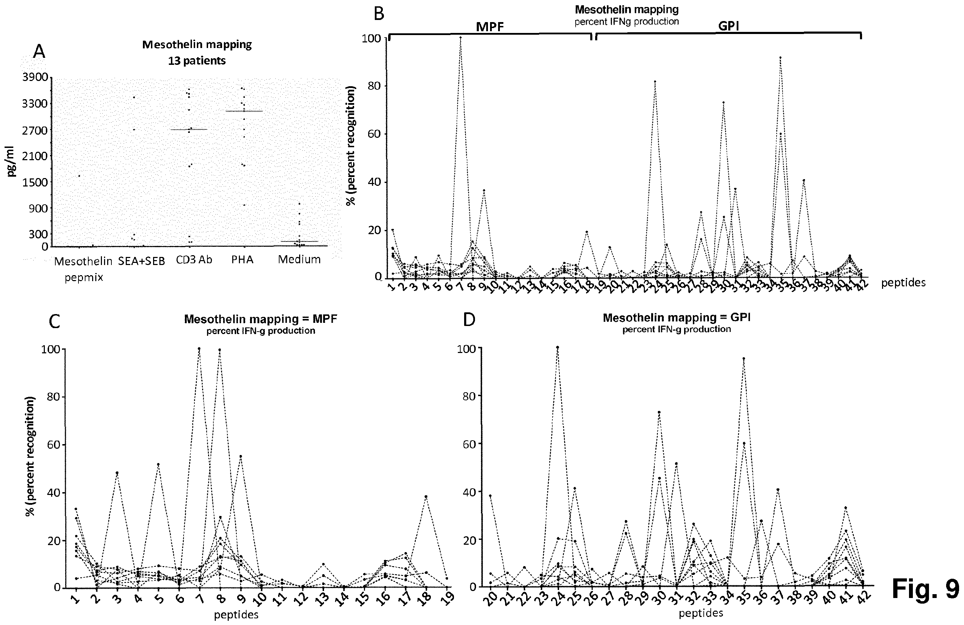

**Fig. 9** shows measured whole-blood IFN-$\gamma$ responses to MPF and GPI-anchored component peptides for mesotheline peptide mapping. (A) absolute IFN-$\gamma$ production in 13 patients treated with the mesothelin peptide mix and controls. (B, C and D) relative IFN-$\gamma$ production upon treatment with single peptides corresponding to the MPF and GPI-anchored component.

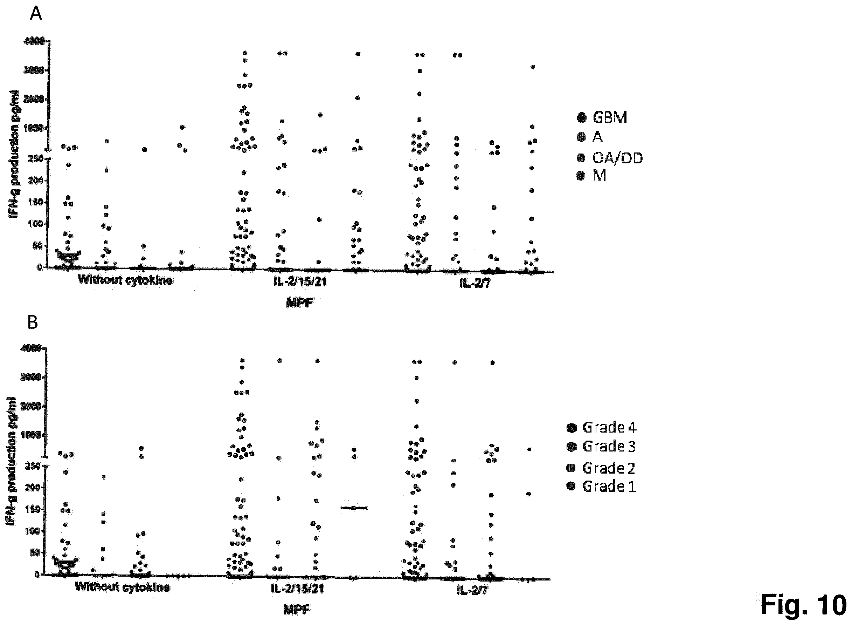

**Fig. 10** shows whole-blood IFN-$\gamma$ responses of glioma patients to MPF component with or without cytokine conditioning. Whole-blood obtained from patients with glioma were cultured with MPF component in the absence of cytokine conditioning, with IL-2/IL-7, or IL-2/IL-15/IL-21 conditioning over seven days. Responses are shown based on (A) histological grading and (B) WHO tumour grading.

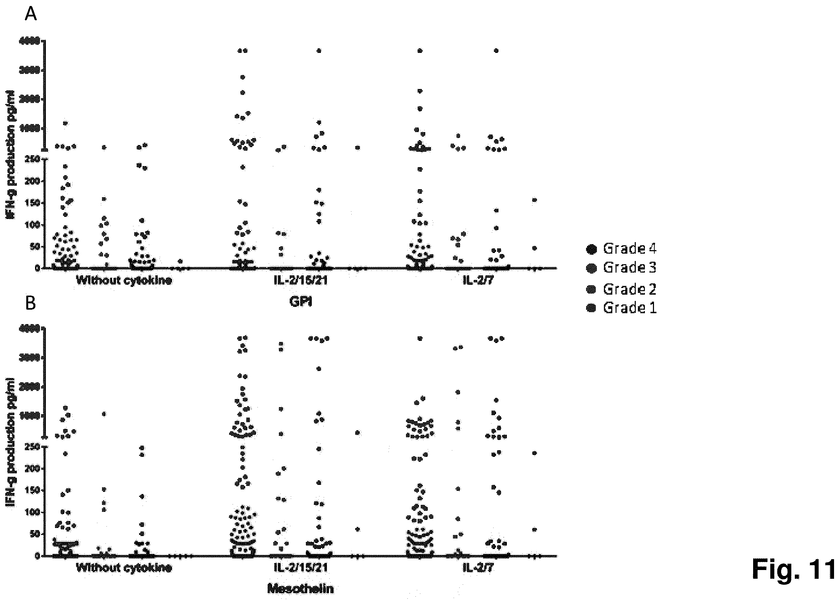

**Fig. 11** shows whole-blood IFN-$\gamma$ responses of glioma patients to (A) GPI-anchored component and (B) full-length mesothelin with or without cytokine conditioning. Whole-blood obtained from patients with glioma were cultured with GPI-anchored component and (B) full-length mesothelin in the absence of cytokine conditioning, with IL-2/IL-7, or IL-2/IL-15/IL-21 conditioning over seven days. Responses are shown based on WHO tumour grading.

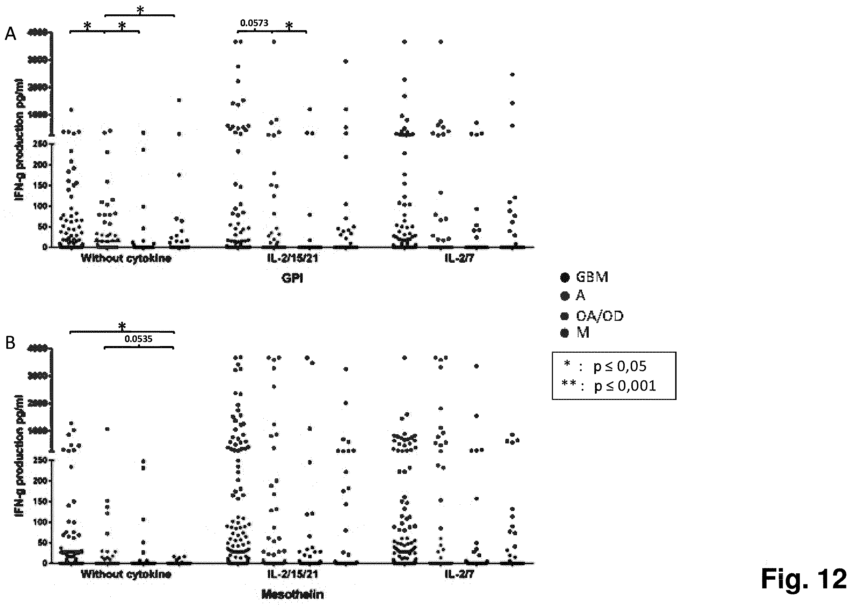

**Fig. 12** shows whole-blood IFN-$\gamma$ responses of glioma patients to (A) GPI-anchored component and (B) full-length mesothelin with or without cytokine conditioning. Whole-blood obtained from patients with glioma were cultured with GPI-anchored component and full-length mesothelin in the absence of cytokine conditioning, with IL-2/IL-7, or IL-2/IL-15/IL-21 conditioning over seven days. Responses are shown based on histological grading. Statistical significance. *$p<0.05$; **$p<0.001$.

## DETAILED DESCRIPTION OF THE INVENTION

In order to provide a clear and consistent understanding of the specification and claims, and the scope to be given such terms, the following definitions are provided.

### Definitions

The term "**antibody**", as used herein, is intended to refer to immunoglobulin molecules comprised of four polypeptide chains, two heavy (H) chains and two light (L) chains interconnected by disulfide bonds (i.e., "full antibody molecules"), as well as multimers thereof (e.g. IgM).
A "**peptide**" as used herein relates to a chain of amino acids connected by peptide bonds. A peptide may be composed of any number of amino acids of any type, preferably naturally occurring amino acids, which, preferably, are linked by peptide bonds. In particular, a peptide comprises at least 3 amino acids, preferably at least 5, at least 7, at least 9, at least 12, or at least 15 amino acids. Furthermore, there is no upper limit for the length of a peptide. However, preferably, a peptide according to the invention does not exceed a length of 500 amino acids, more preferably it does not exceed a length of 300 amino acids; even more preferably it is not longer than 250 amino acids. Thus, the term "peptide" includes "oligopeptides", which usually refer to peptides with a length of 2 to 20 amino acids and "polypeptides" with at least 60, at least 80, preferably at least 100 amino acids.
The term "**polypeptide**" as used herein refers to a peptide The terms "polypeptide" and "protein" are used interchangeably. The polypeptides and proteins as used herein include chemically synthesized proteins as well as naturally synthesized proteins which are encoded by genes. The polypeptides or proteins may be obtained from a natural source, such as human blood or produced in cell culture as recombinant proteins.
The term "**protein**" as used herein relates to may contain one or more polypeptide chains. Proteins with one and one polypeptide chain are often expressed on one polypeptide chain from one gene and cleaved post translation only. Thus, the terms "protein" and "polypeptide" are often used interchangeably.
The term "**protein domain**" or "**domain**" as used herein refers to a region of a protein that can fold into a stable three-dimensional structure independently of the rest of the protein. This structure may maintain a specific function associated with the domain's function within the intact protein, including enzymatic activity, creation of a recognition motif for another molecule, or provision of the necessary structural components for a protein to exist in a particular environment. Protein domains are usually evolutionarily conserved regions of proteins, both within a protein family and within other protein superfamilies that require similar functions.
As used herein, the term "**epitope**" relates to the exact binding position, in particular the amino acids, of a protein, that is bound by a binding protein such as an antibody or a TCR.
The term "**fusion protein**" according to the invention relates to proteins created through the joining of two or more genes, cDNAs or sequences that originally coded for separate proteins/peptides. The genes may be naturally occurring in the same organism or different organisms or may synthetic polynucleotides.

The term "**therapeutic protein**" as used herein relates to proteins or polypeptides with a therapeutic effect, i.e. proteins used as active pharmaceutical ingredient.

An "**isolated**" nucleic acid molecule is one which is separated from other nucleic acid molecules which are present in the natural source of the nucleic acid molecule. For example, with regards to genomic DNA, the term "isolated" includes nucleic acid molecules which are separated from the chromosome with which the genomic DNA is naturally associated. Preferably, an "isolated" nucleic acid molecule is free of sequences which naturally flank the nucleic acid molecule (i.e., sequences located at the 5' and 3' ends of the nucleic acid) in the genomic DNA of the organism from which the nucleic acid molecule is derived.

According to the invention the terms "**protein precursor**", "**pro-protein**" or "**pro-peptide**", relate to an inactive protein (or peptide) that can be turned into an active form by post-translational modification, enzymatic cleavage of a portion of the amino acid sequence.

The relatedness between two amino acid sequences or between two nucleotide sequences is described by the parameter "**sequence identity**". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the no brief option) is used as the percent identity and is calculated as follows:

(Identical Residues x 100)/(Length of Alignment - Total Number of Gaps in Alignment).

The terms "**similarity**" and "**similar**" as used herein with respect to the definition of a peptide or polynucleotide relate to a specified degree of sequence identity of the amino acid sequence or nucleotide sequence with a reference. A similar amino sequence is taken to include an amino acid sequence that is at least 80 %, 85 %, 86 %, 87 %, 88 %, 89 %, 90 %, 91 %, 92 %, 93 %, 94 %, 95 %, 96 %, 97 %, 98 % or even 99% identical to the subject sequence, using the conventional sequence alignment tool Clustal V with default parameters. Typically, similar sequences will include the same residues in positions that are relevant for the function of the peptide or polynucleotide, such as active site residues or glycosylated amino acids, however though may include any number of conservative amino acid substitutions.

"**Identical**" as used herein refers to an amino acid or nucleotide sequence identity to a reference sequence of 100 %.

The term "**recombinant**" when used in reference to a cell, nucleic acid, protein or vector, indicates that the cell, nucleic acid, protein or vector has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell, or express native genes at different levels or under different conditions than found in nature.

As used herein, the term "**immune cell**" includes cells that are of hematopoietic origin and that play a role in the immune response. Immune cells include lymphocytes, such as B cells and T cells; natural killer cells; myeloid cells, such as monocytes, macrophages, eosinophils, mast cells, basophils, and granulocytes.

As used herein, the term "**T cell**" includes cells bearing a T cell receptor (TCR). Preferably, the term "T cell" includes CD4+ T cells and/or CD8+ T cells. The term T cell also includes both T helper 1 type T cells and T helper 2 type T cells. In one embodiment, a T cell of the invention is a naïve T cell, i.e., not an activated or memory T cell. In one embodiment, a T cell of the invention is an activated or memory T cell. These cells can be distinguished using cell markers known in the art. For example, activated T cells express markers such as CD152 and CD154. Activated T cells also can be characterized by their enhanced ability to produce cytokines, proliferate, or perform certain effector functions.

The term "**antigen presenting cell**" includes professional antigen presenting cells (e.g., B lymphocytes, monocytes, dendritic cells, Langerhans cells) as well as other antigen presenting cells (e.g., keratinocytes, endothelial cells, astrocytes, fibroblasts, oligodendrocytes).

A "**chimeric antigen receptor (CAR)**" as used herein is an artificially constructed hybrid protein or polypeptide containing the antigen binding domain of an antibody (e.g., single chain variable fragment (scFv)) linked to T-cell signaling domains. Characteristics of CARs include their ability to redirect T-cell specificity and reactivity toward a selected target in a non-MHC-restricted manner, exploiting the antigen-binding properties of monoclonal antibodies. The non-MHC-restricted antigen recognition gives T cells expressing CARs the ability to recognize antigen independent of antigen processing, thus bypassing a major mechanism of tumor escape. Moreover, when expressed in T-cells, CARs advantageously do not dimerize with endogenous T cell receptor (TCR) alpha and beta chains.

"**Tumor associated antigens**" or "**TAA**" according to the invention are antigens that are presented by MHC I or MHC II molecules or non-classical MHC molecules on the surface of tumor cells. As used herein TAA includes "tumor-specific antigens" which are found only on the surface of tumor cells, but not on the surface of normal cells.

The term "**patient**" as used herein denotes a mammal, in particular a human with brain cancer, who is to be treated.

As used herein, the term "**mammal**" refers to any mammal, including, but not limited to, mammals of the order Rodentia, such as mice and hamsters, and mammals of the order Logomorpha, such as rabbits, Carnivora, including Felines (cats) and Canines (dogs), Artiodactyla, including Bovines (cows) and Swines (pigs) or of the order Perssodactyla, including Equines (horses), Primates, Ceboids, or Simoids (monkeys) or of the order Anthropoids (humans and apes).

"**Expansion**" or "clonal expansion" as used herein means production of daughter cells all arising originally from a single cell. In a clonal expansion of lymphocytes, all progeny share the same antigen specificity.

As used herein, "**interleukin 2**" or "IL-2" refers to human IL-2 as defined by accession number P60568 of the UniProtKB database (http://www.uniprot.org/uniprot/) and functional equivalents thereof. Functional equivalents of IL-2 include relevant substructures or fusion proteins of IL-2 that remain the functions of IL-2. Accordingly, the definition IL-2 comprises any protein with a sequence identity to UniProtKB P60568-1of at least 80 %, preferably at least 90 %, more preferably at least 95 %, most preferably at least 98 %. Recombinant human IL-2 produced in *E. coli* as a single, non-glycosylated polypeptide chain with 134 amino acids and having a molecular mass of 15 kDa is commercially available in lyophilized form from Prospec as CYT-209.

As used herein, "**interleukin 15**" or "IL-15" refer to human IL-15 as defined by accession number P40933 of the UniProtKB database and functional equivalents thereof. Functional equivalents of IL-15 include relevant substructures or fusion proteins of IL-15 that remain the functions of IL-15. Accordingly the definition IL-15 comprises any protein with a sequence identity to UniProtKB P40933-1 of at least 80 %, preferably at least 90 %, more preferably at least 95 %, most preferably at least 98 %. Recombinant human IL-15 produced in *E. coli* as a single, non-glycosylated polypeptide chain with 114 amino acids (and an N-terminal Methionine) and having a molecular mass of 12.8 kDa is commercially available in lyophilized form from Prospec as CYT-230.

As used herein, "**interleukin 21**" or "IL-21" refer to human IL-21 as defined by accession number Q9HBE4 of the UniProtKB database and functional equivalents thereof. Functional equivalents of IL-21 include relevant substructures or fusion proteins of IL-21 that remain the functions of IL-21. Accordingly the definition IL-21 comprises any protein with a sequence identity to UniProtKB Q9HBE4-1 of at least 80 %, preferably at least 90 %, more preferably at least 95 %, most preferably at least 98 %. Recombinant human IL-21 produced in *E. coli* as a single, non-glycosylated polypeptide chain with 132 amino acids and having a molecular mass of 15 kDa is commercially available in lyophilized form from Prospec as CYT-408. The transitional term "**comprising**", which is synonymous with "including," "containing," or "characterized by," is inclusive or open-ended and does not exclude additional, unrecited elements or method steps. The transitional phrase "consisting of" excludes any element, step, or ingredient not specified in the claim, except for impurities ordinarily associated therewith. When the phrase "**consists of**" appears in a clause of the body of a claim, rather than immediately following the preamble, it limits only the element set forth in that clause; other elements are not excluded from the claim as a whole. The transitional phrase "consisting essentially of" limits the scope of a claim to the specified materials or steps "and those that do not materially affect the basic and novel characteristic(s)" of the claimed invention. "A 'consisting essentially of" claim occupies a middle ground between closed claims that are written in a 'consisting of' format and fully open claims that are drafted in a 'comprising' format."

## Mesothelin Binding Protein

Mesothelin is a 40 kDa tumour differentiation antigen present on normal mesothelial cells. The mesothelium is a membrane composed of simple squamous epithelium [15] that forms the lining of several body cavities: the pleura (thoracic cavity), peritoneum (abdominal cavity including the mesentery), mediastinum and pericardium (heart sac). Mesothelial tissue also surrounds the male internal reproductive organs (the tunica vaginalis testis) and covers the internal reproductive organs of women (the tunica serosa uteri). Mesothelium that covers the internal organs is called visceral mesothelium, while the layer that covers the body walls is called the parietal mesothelium. Mesothelium is the epithelial component of serosa.

Full-length, unprocessed mesothelin comprises two components, namely the 31 kDa megakaryocyte-promoting factor (MPF) and the membrane-anchored, 40 kDa mesothelin-glycoinositolphoslipid (GPI) component [8]. MPF is cleaved by furin and shed into systemic circulation, and has been evaluated as a more accurate biomarker than full-length mesothelin for the immunodiagnosis of mesothelioma [11].

The amino acid sequence of human mesothelin precursor is

```
MALPTARPLLGSCGTPALGSLLFLLFSLGWVQPSRTLAGETGQEAAPLDGVLANPPNISS
LSPRQLLGFPCAEVSGLSTERVRELAVALAQKNVKLSTEQLRCLAHRLSEPPEDLDALPL
DLLLFLNPDAFSGPQACTRFFSRITKANVDLLPRGAPERQRLLPAALACWGVRGSLLSEA
DVRALGGLACDLPGRFVAESAEVLLPRLVSCPGPLDQDQQEAARAALQGGGPPYGPPSTW
SVSTMDALRGLLPVLGQPIIRSIPQGIVAAWRQRSSRDPSWRQPERTILRPRFRREVEKT
ACPSGKKAREIDESLIFYKKWELEACVDAALLATQMDRVNAIPFTYEQLDVLKHKLDELY
PQGYPESVIQHLGYLFLKMSPEDIRKWNVTSLETLKALLEVNKGHEMSPQAPRRPLPQVA
TLIDRFVKGRGQLDKDTLDTLTAFYPGYLCSLSPEELSSVPPSSIWAVRPQDLDTCDPRQ
LDVLYPKARLAFQNMNGSEYFVKIQSFLGGAPTEDLKALSQQNVSMDLATFMKLRTDAVL
PLTVAEVQKLLGPHVEGLKAEERHRPVRDWILRQRQDDLDTLGLGLQGGIPNGYLVLDLS
MQEALSGTPCLLGPGPVLTVLALLLASTLA  (SEQ ID NO: 1)
```

Cell membrane-bound (or mature) mesothelin selectively binds to mucin 16 (MUC16), which is expressed in the peritoneum, pleural cavities, mucosal surfaces and the brain [12]. In addition, a 2014 study showed that brain metastasis is highly likely in patients with triple negative breast carcinoma who are mesothelin positive at diagnosis (63%) [13]. Mesothelin overexpression in approximately 50% of advanced gastroesophageal cancers was recently reported, where expression levels were shown to increase with tumor staging [14].

Tumor or cancer cells can over-express mesothelin or express mesothelin at a significantly higher level, as compared to the expression of mesothelin by normal, non-tumor, or non-cancerous cells. Mesothelin is over-expressed by tumor or cancer cells from a variety of different cancers such as, e.g., ovarian cancer, pancreatic cancer, lung cancer (e.g., lung adenocarcinoma), esophageal cancer, gastric cancer, synovial sarcoma, and mesothelioma (US 2015 0031624 and [8-10])

The inventors have identified for the first time an over expression of mesothelin in human brain cancer cells, i.e. GBM tissue cells (see Example 1). have identified specific epitope regions in the GBM expressed mesothelin, which are particularly eligible for targeting by immune therapeutical agents such as anti-mesothelin antibodies or mesothelin binding TCRs (see Example 5).

Mesothelin is a known target for the treatment of cancers such as ovarian cancer, pancreatic cancer, and lung cancer, in which it was known to be over-expressed. For these types of cancers, specific antibodies, antibody-drug conjugates and CAR T-cells have been developed.

However, an expression of mesothelin in brain cancer cells, in particular GBM tissue cells is unexpected. All of the tumor cells, in which an overexpression of mesothelin was found before, were part of tissues derived either from the endoderm or the mesoderm. In contrast, cells forming the central nervous system including the brain are derived from the ectoderm. During embryogenesis, neural stem cells (NSCs) develop from neuroepithelial stem cells which are derived from ectodermal stem cells.

As such, it is generally accepted that GBM arises from an own type of cancer stem cells, namely glioma stem-like cells (GSCs), which in turn may be derived from either directly from NSCs, or further differentiated neuronal cells such as neural progenitor cells or differentiated neuronal cells, e.g. astrocytes [16].

Due to the novel findings, approaches of targeting mesothelin can now be used in the treatment of patients with brain cancer.

Thus, according to a first aspect, the present invention relates to a mesothelin binding protein for use in the treatment of brain cancer in a patient, wherein said mesothelin binding protein comprises an epitope binding domain, which specifically binds to an epitope containing amino acid sequence that is similar or identical to a section of the amino acid sequence of human mesothelin precursor (SEQ ID NO: 1).

The brain cancer to be treated can be any type of brain cancer. In particular, the brain cancer can be a brain cancer of grades I-IV. Examples of brain cancers are glioma, astrocytoma, juvenile pilocytic astrocytoma, low grade astrocytoma, anaplastic astrocytoma, glioblastoma, oligodendroglioma, and ependymoma. According to one embodiment of the first aspect, the brain cancer is GBM.

It is assumed that mesothelin is not only overexpressed in human brain cancers but also in brain cancers of other in mammalians. Thus, the patient to be treated can be selected from a rodent, a feline, a canine and a primate. Preferably, the patient selected from a human, a dog or a horse.

The reference mammalian mesothelin is dependent on the patient to be treated. For example, dogs and horses express mesothelin-like proteins identified by UnitprotKB database entries F1 PFV8 and F6WA23, respectively.

The treatment is preferably an mesothelin directed immunotherapy. Cancer immunotherapies can be either passive or active. Passive therapy is based on the adoptive transfer of immunomodulators including cytokines, tumor specific antibodies, antibody drug conjugates or immune cells such CAR T-cells. These substances or cells are then administered to the patient to initiate an anti-tumor action. Active immunotherapies, on the other hand, stimulate the patient's immune system with the intent of promoting an antigen specific anti-tumor effect using the body's own immune cells, e.g. the expansion of autologous T-cells.

The epitope bound by the mesothelin binding protein may be located anywhere in the mesothelin precursor, i.e. either in the GPI-anchored to mature mesothelin (GPI) or the megakaryocyte-promoting factor (MPF). Thus according to one embodiment the section of the amino acid sequence of the human mesothelin precursor is located within the MPF or GPI. As shown in the examples, both parts of the mesothelin precursor were found to contain and are therefore eligible for targeting by mesothelin binding proteins for treatment. The immune recognition hotspots of MPF are summarized in the following Table 1.

Table 1: Peptide sequences

| Peptide | Amino acid sequence | Identifier |
| --- | --- | --- |
| P1 | MALPTARPLLGSCGT | Amino acids 1- 15 of SEQ ID NO:1 |
| P8 | HRLSEPPEDLDALPL | Amino acids 106 - 120 of SEQ ID NO:1 |
| P24 | YEQLDVLKHKLDELY | SEQ ID NO:2 |
| P32 | WAVRPQDLDTCDPRQ | SEQ ID NO:3 |
| P33 | LDVLYPKARLAFQNM | SEQ ID NO:4 |
| P40 | LQGGIPNGYLVLDLS | SEQ ID NO:5 |
| P41 | MQEALSGTPCLLGP | SEQ ID NO:6 |

However, in contrast to MPF which is generally removed from the cell membrane after cleavage, the GPI remains on the surface of the cells. Accordingly, a mesothelin binding protein targeting an epitope in the GPI is more likely to have a beneficial effect in the treatment of brain cancer.

According to one embodiment of the first aspect, the section of the amino acid sequence of the human mesothelin precursor is located within the GPI anchored mature mesothelin (GPI).

The epitope may be a folding epitope or a sequence epitope. The sequence epitope consists of a specific number of consecutive amino acids in the amino acid sequence of mesothelin which are bound simultaneously by the mesothelin binding protein. A folding epitope consist of a specific number of amino acids that are in close proximity in mesothelin after folding and are contacted at the same time in the event of binding of the mesothelin binding protein. Using peptides with a length of 15 amino acids, the inventors have identified certain specific sequence epitopes. Therefore, the epitope is preferably a sequence epitope. In this regard, the epitope may have a length a length in the range from 2 to 10 amino acids, preferably in the range from 3 to 7 amino acids.

As shown in the examples, the recognition hotspots, including the peptides P24, P32, P33, P40, and P41 in mesothelin are mainly located towards at the C-terminus of the GPI. Therefore, according to one embodiment of the first aspect the section of the amino acid sequence of the human mesothelin precursor starts after amino acid 345 of SEQ ID NO: 1. The sequence of amino acids 466-630 of the mesothelin precursor includes four out of five immune recognition sites. Thus, preferably the section of the amino acid sequence of the human mesothelin precursor starts after amino acid 465 of SEQ ID NO: 1.

The immune recognition epitopes according to the examples are in particular located within the peptide sequences SEQ ID NO: 2, SEQ ID NO: 3, SEQ ID NO: 4, SEQ ID NO: 5, SEQ ID NO: 6.

A naturally occurring mesothelin binding protein is Muc16, also known as CA125.

Muc16 binds to GPI anchored mesothelin in the range from amino acid 265-359. Accordingly, the mesothelin binding protein may comprise Muc16 or fragment thereof. Such fragment must contain the mesothelin binding domain of Muc16 or a structural variant thereof. The Muc16 or fragment thereof may be part of a recombinant protein, such as a fusion protein containing further functional parts. The mesothelin binding protein comprising Muc16 or fragment thereof may be conjugated to an anti-cancer drug.

**Anti-Mesothelin Antibodies**

Alternatively, according to one embodiment the mesothelin binding protein comprises at least a fragment of an antibody. Antibodies recognizing mesothelin may e.g. act via antibody-dependent cell-mediated cytotoxicity (ADCC).

Antibodies and antibody fragment according to the invention includes an epitope binding domain of an antibody. The term "epitope binding domains", as used herein, refers to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region;

8

(iii) a Fd fragment consisting of the VH and CH1 domains; (iv) a Fv fragment consisting of the VL and VH domains of a single arm of an antibody, (v) a dAb fragment ([21], which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g. [22], [23], [24]). Such single chain antibodies are also intended to be encompassed within the term "antigen-binding portion" of an antibody. Any VH and VL sequences of specific scFv can be linked to human immunoglobulin constant region cDNA or genomic sequences, in order to generate expression vectors encoding complete IgG molecules or other isotypes. VH and VL can also be used in the generation of Fab, Fv or other fragments of immunoglobulins using either protein chemistry or recombinant DNA technology. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., [25], [26])

Preferred antibody fragments are fragments that contain epitope binding domains. Antibody fragments containing an epitope binding domain are selected from the group consisting of a Fab fragment, a F(ab')$_2$ fragment, a Fd fragment, a Fv fragment, a dAb fragment, an isolated complementarity determining region (CDR) and an single chain Fv (scFv). There are several mesothelin binding antibodies and antibody fragments described in the art.

The mesothelin binding antibody may be a chimeric monoclonal antibody. Also, the mesothelin binding antibody may be a humanized antibody.

An example of the mesothelin binding antibody is MORAb-009. MORAb-009 is a high-affinity chimeric (mouse/human) monoclonal IgG1/κ with high affinity and specificity for mesothelin [17]. An example of a mesothelin binding antibody fragment is anti-mesothelin Fv of SS1P. The anti-mesothelin FV of SS1P is it described in [18]

The mesothelin binding protein may be a recombinant protein. In particular, the mesothelin binding protein comprising an antibody or fragment thereof maybe a fusion protein containing additional functional parts, i.e. peptide sequences. Moreover, according to one embodiment, the mesothelin binding protein is conjugated to an anti-cancer drug. For example, conjugated monoclonal antibodies are joined to a drug, which is either cytotoxic or radioactive. The toxic chemicals are those typically used as chemotherapy drugs, but other toxins can be used. As the mesothelin binding protein or antibody binds to specific antigens on cancer cell surfaces it directing the drug to the tumor. Radioactive compound-linked proteins or antibodies are referred to as radiolabelled. Chemolabelled or immunotoxins antibodies are tagged with chemotherapeutic molecules or toxins, respectively.

Examples of anti-cancer drugs are DNA-alkylating agents such as duocarmycin or MDX-1382, tubulin polymerase inhibitors such as DM4 or BAY 94-9343, a pseudomonas toxin, a cytokine such as IL-12, or a radioactive substance, and cisplatin.

According to one embodiment, the anti-cancer drug conjugated to the mesothelin binding protein is a selected from the group consisting of a pseudomonas toxin, a cytokine such as IL-12, or a radioactive substance, and cisplatin.

An example of an anti-mesothelin antibody-drug conjugate that may be used is SS1P. SS1P is a recombinant immunotoxin consisting of an anti-mesothelin Fv-linked to a truncated *Pseudomonas* exotoxin that mediates cell killing [18, 19].

## CAR T-cells

Moreover, a different approach of immunotherapy targeting mesothelin in brain cancer is in the use of CAR T-cells which specifically bind to mesothelin. Thus, according to an alternative embodiment, the mesothelin binding protein is a chimeric antigen receptor (CAR). In this regard, the CAR is preferably a part of a CAR T-cell. The manufacturing of CAR T-cells is known in the art and for example described in [38], which is incorporated herein by reference.

Examples are "Autologous chimeric immune receptor (CIR) T-cells" transfected with anti-mesothelin chimeric T-cell receptor mRNA, with potential antineoplastic activity. The anti-mesothelin mRNA encodes a single chain antibody variable fragment (ScFv), the intracellular CD 3 zeta T-cell receptor domain and the 4-1BB (cd137) (NCI Thesaurus Code: C97038).

## Expanded T-cells

Further immunotherapy involves the use of T-cells specific for TAA such as mesothelin. Therefore, according to one embodiment, the mesothelin binding protein is a T-cell receptor (TCR).

In this regard, the mesothelin binding protein, i.e. TCR will be used in the form of an expanded T-cell in which the TCR is expressed and presented on the cell membrane.

Thus according to a second aspect, the invention provides an expanded T-cell for use in the treatment of brain cancer in a patient, wherein said T-cell comprises a mesothelin binding TCR according to the first aspect.

The mesothelin targeting T-cell is preferably part of a population of expanded T-cells. According to a preferred embodiment of the second aspect, the T-cell is manufactured by clonal expansion of T-cells by culturing of a T-cell containing sample in the presence of a cytokine cocktail comprising at least two of interleukin 2 (IL-2), interleukin 15 (IL-15) and interleukin 21 (IL-21) (as described in WO 2015/189357 A1).

According to one embodiment, the T-cell containing sample is a body sample previously obtained from a patient. Preferably, the body sample is an autologous body sample of the patient to be treated with the mesothelin targeting T-cell.

The body sample can be selected from tumor, blood, tears, saliva, synovial fluid, placenta tissue, bone marrow, liquor and ascites.

A body sample derived from tumor is also referred as tumor infiltrating lymphocytes (TIL). TIL is any kind of lymphocyte that is located in, on or around a tumor. Due to their localization in the tumor, the TIL may have experienced tumor-associated antigens. Accordingly, mesothelin targeting T-cells, can be expanded with the method according to the invention without expansion antigen.

A sample from peripheral blood is also referred to peripheral blood mononuclear cells (PBMCs). Depending on the type of disease different body samples may be preferred.

Culturing of the body sample *in vitro* to expand the T-cells may comprise one or more sub-steps. Accordingly, in one embodiment the *in vitro* culturing comprises a first expansion step comprising incubation in culture medium comprising IL-2, IL-15 and IL-21 until lymphocytes become detectable

"Detectable" according to the invention means that the lymphocytes, for example, become visible, in particular by microscopy. T-cells are usually become detectable using standard light microscopy upon reaching a concentration of $5 \times 10^3$ cells/ml.

The detection of the T-cells may include any method known in the art that is eligible to detect the presence of lymphocytes above a certain threshold. The first expansion step has the purpose of gently inducing cell proliferation together with a stimulation of the cells by the cytokine cocktail.

The time of incubation of the first expansion step is in the range from 6 hours to 180 days. For example, lymphocytes derived from glioblastoma are for example detectable after one to two weeks.

According to one embodiment of the invention, the culture medium of the first expansion step comprises at least one expansion antigen. In one embodiment of the invention the culture medium of the first expansion step comprises multiple expansion antigens.

The expansion antigen is preferably a mesothelin peptide that has an amino acid sequence identical or similar to a section of the amino acid sequence of the human mesothelin precursor (SEQ ID NO: 1).

As shown in the examples, the immunization hotspots were found in the peptides P24, P32, P33, P40, and P41 which represent mesothelin sections located towards at the C-terminus of the GPI. Moreover, these immunization hotspots showed strong IFN-γ responses when tested in the expansion of autologous patient T-cells. Thus, preferably, the section starts after amino acid 345 of SEQ ID NO: 1.

According to one embodiment, the section of the amino acid sequence of the human mesothelin precursor starts after amino acid 345 of SEQ ID NO: 1. The sequence of amino acids 466-630 of the mesothelin precursor includes four out of five immune recognition sites. Thus, preferably the section of the amino acid sequence of the human mesothelin precursor starts after amino acid 465 of SEQ ID NO: 1.

Preferably, the amino acid sequence has an identity of 90%, 95%, 98%, 99%, or 100% to the section human mesothelin precursor. In order to minimize cross-reaction with healthy tissue it is preferred to use an expansion antigen which is not exactly identical to the mesothelin sequence but contains at least one mutation relative to the sequence human wild-type mesothelin..

The expansion antigen may have an amino acid sequence with an identity of 90%, 95%, 98%, 99%, or 100% to SEQ ID NO: 2. Alternatively, the expansion antigen may have an amino acid sequence with an identity of 90%, 95%, 98%, 99%, or 100% to SEQ ID NO: 3. Moreover, the expansion antigen may have an amino acid sequence with an identity of 90%, 95%, 98%, 99%, or 100% to SEQ ID NO: 4, Furthermore, the expansion antigen may have an amino acid sequence with an identity of 90%, 95%, 98%, 99%, or 100% to SEQ ID NO: 5, Preferably, the expansion antigen has an amino acid sequence with an identity of 90%, 95%, 98%, 99%, or 100% to SEQ ID NO: 6.

The first expansion step may be followed by a second expansion step wherein the cells are incubated with feeder cells and/or an antibody against CD3 in addition to the at least two types of cytokines selected from IL-2, IL-15 and IL-21. An expansion with feeder cells and the antibody against CD3 has been described in the state of the art. It is believed that feeder cells lead to an improvement of cell growth. Feeder cells are irradiated cells that do not proliferate or proliferate only to a small extent. The feeder cells increase the number of cell contacts in the culture and additionally feed the proliferating and expanding cell culture. Feeder cells are preferably in irradiated PBMCs. Allogenic feeder cells are from a different organism as the mammal to be treated with the expanded clinically relevant lymphocytes. Autologous feeder cells are from the mammal to be treated.

The antibody against CD3 is preferably the antibody defined as OKT3. OKT3 is a murine monoclonal antibody of the immunoglobulin IgG2a isotype. The target of OKT3, CD3, is a multi-molecular complex found only on mature T-cells. An interaction between T-cells, OKT3 and monocytes causes T-cell activation in vitro.

Preferably, feeder cells are used in combination with CD3 and the cytokines IL-2, IL-15 and IL-21. According to one embodiment of the method according to the second aspect, the ratio of feeder cells to lymphocytes is in the range from 1:1 to 1:100. Preferably, the ration of feeder cells to lymphocytes is in the range from 1:2 to 1:50. As shown in the examples very low ratios of feeder cells are sufficient for a strong expansion of clinically relevant lymphocytes, in particular clinically relevant T-cells.

In the examples, a ratio of 1:10 is sufficient to support growth and expansion of lymphocytes. Accordingly it is believed that a value in the range from 1:5 to 1:20 would not lead to a different result. The low number of feeder cells has at least two advantages. First, there are less distracting cellular signals allowing more homogeneous and reliable expansion results. Secondly, fewer feeder cells lead to less exogenous material in the immunotherapy product obtained by the method, i.e. the population of the clinically relevant lymphocytes.

The second expansion step optionally also includes an expansion antigen in the culture medium. Preferably no clinically relevant antigen or fragment is added to the medium of the second expansion step.

## Cancer Vaccine

A further established cancer immunotherapy is the use of cancer vaccines. There are two broad types of cancer vaccine. Preventive (or prophylactic) vaccines, which are intended to prevent cancer from developing in healthy people and treatment (or therapeutic) vaccines, which are intended to treat an existing cancer by strengthening the body's natural immune response against the cancer. Treatment vaccines for immunotherapy are preferred. The cancer vaccines are usually based on tumor associated antigens.

Thus according to a third aspect of the invention provides a cancer vaccine in the treatment of a brain cancer in a patient, wherein the cancer vaccine is selected from a vaccine peptide comprising an amino acid sequence that is similar or identical to the amino acid sequence of the human mesothelin precursor or a fragment thereof, or a nucleotide encoding said vaccine peptide.

One example of a mesothelin targeting cancer vaccine is CRS-207, and attenuated form of *Listeria monocytogenes* vector that overexpresses human mesothelin [20], which is tested in clinical studies for the treatment of pancreatic cancer and ovarian cancer. Based on the present findings this vaccine can now be used for the treatment of brain cancer, in particular GBM.

Based on the experimental results according to the examples the peptides P24, P32, P33, P40, and P41 of mesothelin are recognition hotspots. these peptides were not only frequently recognized by serum antibodies but also showed strong IFN-$\gamma$ responses in the expansion of patients' autologous was T cells.

According to one embodiment of the third aspect, the vaccine peptide has an amino acid sequence similar or identical to the amino acid sequence of a fragment of the human mesothelin precursor starting after amino acid 345 of SEQ ID NO: 1.

The sequence of amino acids 466-630 of the mesothelin precursor includes four out of five immune recognition sites. Thus, preferably the vaccine peptide has an amino acid sequence similar or identical to the amino acid sequence of a fragment of the human mesothelin precursor starting after amino acid 465 of SEQ ID NO: 1.

More preferably, the amino acid sequence over the vaccine protein has an identity of 90%, 95%, 98%, 99%, or 100% to the fragment human mesothelin precursor. In order to minimize cross-reaction with healthy tissue it is preferred to use a vaccine peptide which is not exactly identical to the mesothelin sequence but contains at least one mutation relative to the sequence human wild-type mesothelin.

The expansion antigen may have an amino acid sequence with an identity of 90%, 95%, 98%, 99%, or 100% to SEQ ID NO: 2. Alternatively, vaccine peptide may have an amino acid sequence with an identity of 90%, 95%, 98%, 99%, or 100% to SEQ ID NO: 3. Moreover, the vaccine peptide may have an amino acid sequence with an identity of 90%, 95%, 98%, 99%, or 100% to SEQ ID NO: 4, Furthermore, the vaccine peptide may have an amino acid sequence with an identity of 90%, 95%, 98%, 99%, or 100% to SEQ ID NO: 5, Preferably, the vaccine peptide has an amino acid sequence with an identity of 90%, 95%, 98%, 99%, or 100% to SEQ ID NO: 6.

According to one embodiment, the cancer vaccine is a polynucleotide encoding the vaccine peptide defined above. The polynucleotide may be selected from an RNA or a DNA molecule.

The polynucleotide may be an isolated polynucleotide or part of a vector. Several vectors for cancer vaccines have been described in the art, for example based on Adenovirus, Venezuelan equine encephalitis virus (VEE), Sindbis virus (SIN), Semliki forest virus (SFV), and VEE-SIN chimeras, or Pox viruses, reviewed in [31], which is incorporated herein by reference in its entirety. In addition, vectors based on *Listeria monocytogenes* may be used.

**Administration**

To practice the therapeutic uses or methods disclosed herein, an effective amount of the mesothelin binding protein, the CAR T-cell, the expanded T-cell or the cancer vaccine, can be administered to a subject (e.g., a human cancer patient, a patient with an infectious disease, a patient with an autoimmune disease) in need of the treatment via a suitable route, such as, for example, intravenous administration. The cells may be introduced by injection, catheter, or the like. If desired, additional drugs (e.g., cytokines) may also be co-introduced or introduced sequentially. Any of the cells or compositions thereof may be administered to a subject in an effective amount. As used herein, an effective amount refers to the amount of the respective agent (e.g., the cells or compositions thereof) that upon administration confers a desirable therapeutic effect on the subject. Determination of whether an amount of the cells or compositions described herein achieved the desired therapeutic effect would be evident to one of skill in the art. For example, see references 2-5. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. In some embodiments, the effective amount alleviates, relieves, ameliorates, improves, reduces the symptoms, or delays the progression of the desired disease or disorder in the subject.

The administering of mesothelin binding protein, the CAR T-cell, or the expanded T-cell preferably is an introduction into the patient either locally in proximity of a tumor or into the tumor. Alternatively mesothelin binding protein, the CAR T-cell, or the expanded T-cell is administered into the blood circuit.

The cancer vaccine is preferably added into the blood circuit, either by subcutaneous or intravenous administration.

**Method of Diagnosis**

Moreover, as shown in the examples, the level of IFN-$\gamma$ response to mesothelin peptides is indicative for the grade of brain cancer. A strong IFN-$\gamma$ response correlates with brain cancer grades III and IV. Thus, according to a fifth aspect, the invention provides a method for diagnosing a patient suspected of a brain tumor, comprising the steps of

a. Providing a fluid body from the patient;
b. Culturing the cells in the fluid body sample in the presence of a cytokine cocktail comprising at least two cytokines selected from IL-2, IL-15 and IL-21, and a mesothelin peptide comprising an amino acid sequence that is similar or identical to the amino acid sequence of the human mesothelin precursor or a fragment thereof to form a culture;
c. Measuring the IFN-$\gamma$ concentration in the culture; and
d. Comparing the concentration of IFN-$\gamma$ to an IFN-$\gamma$ threshold;

In this method, a concentration of IFN-$\gamma$ above the IFN-$\gamma$ threshold is indicative for a Grade III or IV glioblastoma.

The fluid body sample may be selected from a blood sample. The fluid body sample is preferably a blood sample. The fluid blood sample is preferably previously obtained, i.e. the provision of the blood sample does not include the blood withdrawal.

The mesothelin peptide may be similar or identical to a section of the MPF or the GPI as shown in Example 2.

The mesothelin peptide may have an amino acid sequence with an identity of 90%, 95%, 98%, 99%, or 100% to SEQ ID NO: 2. Alternatively, vaccine peptide may have an amino acid sequence with an identity of 90%, 95%, 98%, 99%, or 100% to SEQ ID NO: 3. Moreover, the vaccine peptide may have an amino acid sequence with an identity of 90%, 95%, 98%, 99%, or 100% to SEQ ID NO: 4, Furthermore, the vaccine peptide may have an amino acid sequence with an identity of 90%, 95%, 98%, 99%, or 100% to SEQ ID NO: 5, Preferably, the vaccine peptide has an amino acid sequence with an identity of 90%, 95%, 98%, 99%, or 100% to SEQ ID NO: 6.

The culturing of the sample can be carried out as described in the Example 2.

Furthermore, as shown in the Example 8, further markers for the brain cancer grade are the concentration of mesothelin or fragments thereof and anti-mesothelin antibodies in the patient's blood. In particular, a high concentration of mesothelin

The method for diagnosing may further comprise the steps of:

- Measuring the concentration of mesothelin or fragments thereof in a blood sample if the patient; and
- Comparing the concentration of mesothelin/mesothelin fragments to a mesothelin threshold.

Alternatively, the method for diagnosing may further comprise the steps of:

- Measuring the concentration of anti-mesothelin antibodies in a blood sample if the patient; and
- Comparing the concentration of anti-mesothelin antibodies to an anti-mesothelin antibody threshold.

Of course, the mesothelin and anti-mesothelin antibody determinations can be carried out in parallel and the results combined.

It was found that a concentration of mesothelin/mesothelin fragments above the mesothelin threshold and a concentration of IFNγ above the IFNγ threshold is indicative for a Grade IV glioblastoma. The mesothelin threshold for Grade IV is in particular 10 pg/mL blood.

In case, it is unclear if the patient suffers from a brain cancer or from another type of cancer it is advisable to include a further step for diagnosing brain cancer.

The present invention is further described by the following, non-limiting, examples.

## EXAMPLES

*Patient characteristics/cohort description*

Patients with brain cancer (n = 374) registered at the Karolinska University Hospital, Stockholm were recruited. The patients were diagnosed with the following forms of brain cancer: glioblastoma multiforme (GBM), astrocytoma (A), oligoastrocytoma/ oligodendroglioma (OA/OD) or metastatic brain tumor (Met). All participating patients were chemotherapy-naïve and had not undergone surgery. The tumors were graded according to the WHO grading system for tumors of the central nervous system [27]. The clinical characteristics of the study cohort are summarized in Table 2.

Table 2: Summary of clinical characteristics

| Patient characteristics | Malignant Glioma | | | | | | Metastasis |
|---|---|---|---|---|---|---|---|
| | Histology | | | Grade | | | |
| | GBM | A | OD | IV | III | II | |
| Sample Size(N) | 169 | 45 | 27 | 169 | 20 | 52 | 45 |
| Age Median(Years) | 62 | 34 | 40 | 62 | 48 | 36 | 60 |
| Age Range(Years) | 16-80 | 20-75 | 22-62 | 16-80 | 20-72 | 22-76 | 30-84 |
| Sex(Male/Female) | 111/58 | 33/12 | 14/13 | 111/58 | 13/7 | 32/20 | 21/24 |

*Handling of peripheral blood and tumor tissue obtained from patients*

Peripheral blood samples were collected in heparin-containing tubes by venepuncture and processed in the laboratory within 24 hours. GBM tumor tissue samples were placed in Cell Gro medium (Cell Genix, Freiburg, Germany) containing 10% human AB serum (Innovative Research, Michigan, USA) and processed within 3 hours.

*Statistical analysis*

GraphPad Prism 6 software was used for statistical analysis of differences between patient groups or within each group with the Mann-Whitney U-test or the Wilcoxon test. A p-value of 0.05 or less was considered significant.

**Example 1 - Mature mesothelin is overexpressed in GBM tissue**

The presence of mesothelin in GBM tissue was immunohistologically analyzed. For this purpose, formalin-fixed and paraffin embedded brain tissue sections from patients suffering from GBM were deparaffinised by washing two times, 10 minutes each, in xylene and progressively rehydrated from 100% to 50% ethanol, rinsed in water and placed in PBS for 10 minutes. All washing steps between incubations were performed in PBS. Before blocking and incubation with the primary antibody, tissue sections were subjected to heat-induced epitope retrieval using Antigen Retrieval Reagent-Basic (R&D systems, Minnesota, USA). Sections were washed in PBS and blocked with 1% normal goat serum in PBS for 30 minutes. Incubation with rat monoclonal anti-human mesothelin antibody, 10μg/ml (R&D systems, Minnesota, USA) was performed overnight at 40°C. As a negative control, the primary antibody was omitted and as positive control, pancreatic cancer tumor cell line (PaTu) was used. After washing, tissue sections were incubated for 30 minutes with Alexa Fluor 488-conjugated polyclonal goat anti-rat secondary antibody (Thermo Fisher Scientific, Massachusetts,

USA) at 5μg/ml. Sections were DAPI stained after washing, and mounted in mounting medium containing anti-fade. The stained tissue sections were examined and photographed with a camera-equipped microscope (Olympus BX51, Tokyo, Japan).

Overexpression of mesothelin in GBM tissue could be confirmed in paraffin-embedded tissue sections visualized via fluorescence microscopy (Fig. 1). As a negative control, immunostained tissue sections from the same tissue block with the secondary antibody only was applied. It was observed that the binding of the anti-mesothelin primary antibody was target-specific (**Fig. 1**).

**Example 2 - IFN-γ responses to full-length mesothelin, MPF and GPI-anchored component of whole blood from patients with brain tumor**

In order to assess the induction of typical IFN-γ responses to mesothelin and its derivatives in blood, a whole blood assay was performed. To do so, heparinised whole blood was diluted at a ratio of 1:1.5 with R10 medium (RPMI 1640 L-glutamine (2mM) containing antibiotics (penicillin,100IU/mL and streptomycin, 10mg/mL) (Life Technologies, Carlsbad, USA) and 10% FBS (Life technologies, Carlsbad, USA)) and one of three different cytokine conditions: without cytokines (medium only), IL-7 (10ng/ml) and IL-2 (1000II/ml) or IL-2 (1000IU/ml), IL-15 (10ng/ml) and IL-21 (10ng/ml) (Prospec, Ness-Ziona, Israel). The diluted blood was transferred to a 96-well culture pre-coated with the following antigens: mesothelin peptide pool, megakaryocyte-potentiating factor (MPF) or glycophosphatidylinositol (GPI) anchor for seven days at 37°C with 5% $CO_2$ as described previously ([28] and [29]). Phytohaemagglutinin (PHA) and OKT3 (anti-CD3 monoAb, Biolegend, CA, USA) were used as positive control while medium served as negative control. Supernatants were harvested seven days later to quantify antigen-specific interferon gamma (IFN-γ) production by sandwich ELISA (MABTECH, Stockholm, Sweden).

It was found that conditioning of whole blood from GBM patients with IL-2, IL-15 and IL-21 significantly improved the IFN-γ response to mesothelin peptide pool, as well as the MPF subcomponent (**Fig. 2A**). Compared to absence of cytokine conditioning, conditioning of whole blood with IL-2 and IL-7 gave a stronger IFN-γ response to all three antigens although to a lesser degree than the combination of IL-2, IL-15 and IL-21. The finding was similar for patients with astrocytoma, OA/OD and metastatic brain tumor with regard to IFN-γ response to full-length mesothelin and MPF with IL-2, IL-15 and IL-21 conditioning, respectively (**Fig. 2B-D**). Thus, robust and measurable IFN-γ responses directed against full-length mesothelin and MPF in patients with malignant brain cancer may be amplified by prolonged exposure to IL-2, IL-15 and IL-21.

**Example 3: IFN-γ responses to full-length mesothelin, MPF and GPI-anchored component of whole blood from patients with brain tumor associated to the tumor grade.**

The IFN-γ response data of Example 2 were reanalyzed in relation to the WHO brain tumor grading system, as the severity of disease may affect the immunological fitness of the patient [32], [33]. According to this system, there are four grades of brain tumors in humans: grade I tumors are benign and linked with long-term survival (i.e. pilocytic astrocytoma); grade II tumors are slow-growing with metastatic potential (i.e. low-grade OD and diffuse astrocytoma); grade III tumors are malignant and have a chance to recur at the same grade or progress to grade IV (i.e. OA/OD); grade IV tumors are the most malignant and can metastasize rapidly (exclusively GBM). In the reanalysis, it was found that patients with grade IV brain tumor (GBM and metastatic disease), constituting the majority of the study cohort, had a significantly increased IFN-γ response to MPF and mesothelin peptide pool with IL-2/IL-15/IL-21 conditioning of whole blood (**Fig. 3A**). A similar pattern was also found for patients with grade II brain tumor (**Fig. 3C**). Since very few patients has grade I or III brain tumors, a strong effect of cytokine conditioning on IFN-γ response to the mesothelin antigens could not be seen (**Fig. 3B, D**). Nevertheless, patients with grade III brain tumors may still have a benefit, due to a significant improvement in their IFN-γ response to full-length mesothelin (**Fig. 3B**). It is therefore concluded that the mesothelin-specific response of patients with grade IV brain tumors may be enhanced with IL-2/IL-15/IL-21 conditioning.

**Example 4: FASCIA T-cell proliferation**

In order to investigate whether the patients' IFN-γ production corresponded with enhanced proliferation of T-cells in the WBA, the numbers of CD3+, CD3+CD4+ and CD3+CD8+ cells was measured using flow cytometry. For this purpose, whole blood from GBM patients was treated and incubated as described in Example 2. After seven days of incubation, supernatants from the plates were harvested and stored at -20°C for cytokine detection. The remaining contents of the plate (blood cells) were pooled (for the duplicate wells), washed with phosphate buffered saline (PBS) and stained for the flow cytometric assay for specific cell-mediated immune-response in activated whole blood (FASCIA) [30] with a cocktail of monoclonal antibodies: anti-CD3 FITC, anti-CD4 APC, anti-CD8 PerCP and anti-TCRγδ PE. After 15-minute incubation at 4°C, red blood cells were lysed with Pharm lysing buffer (BD Biosciences, CA, USA) for 10 minutes followed by a 5-minute incubation at room temperature. Cells were then resuspended in PBS and acquired on a FACSCalibur flow cytometer (BD Biosciences, CA, USA). Analysis was done using the FlowJo software (Treestar, OR, USA). The proliferation ratio of the analyzed cells was calculated based on the size and granularity of resting and activated cells (blasts) with the following formula: *PR= blast/(resting cells + blast).* The stimulation percentage (SP%) was defined as a function of the proliferation ratio of the negative (medium) and positive (PHA) controls: $SP\% = (PR_{Ag} - PR_{medim}) / (PR_{PHA} - PR_{medium}) \times 100.$

As a result, conditioning with IL-2/IL-15/IL-21 significantly increased the numbers of total (CD3+), CD4+ and CD8+ T cells in response to full-length mesothelin (**Fig. 4A**). However, compared to IL-2/IL-15/IL-21 conditioning, IL-2/IL-7 addition appeared to have a more pronounced effect on T-cell proliferation in response to PHA stimulation, although both cytokine cocktails markedly improved cell growth (**Fig. 4B**). Thus, conditioning with IL-2/IL-15/IL-21 amplifies the antigen-specific cellular immune response to full-length mesothelin by inducing T-cell proliferation.

### Example 5: Whole-blood IFN-γ response to mesothelin peptides

After confirming the brain cancer patients can mount measurable cellular immune responses to full-length mesothelin, it was analyzed which epitopes within the mesothelin protein evoke the strongest IFN-γ response by T cells. In order to do so, whole blood was diluted 1:1.5 with RPMI and co-incubated in a pre-coated plate with a panel of 42 peptides (1μg/ml) and a mesothelin peptide mix (1μg/peptide/ml; Peptides & Elephants, Potsdam, Germany) at 37°C, 5% $CO_2$ during 7 days. The mesothelin peptide pool is a customized product comprising 42 x 15-mer peptides without overlap covering the entire length of the mesothelin protein. The first 18 peptides comprise the MPF component, while the following 24 peptides constitute the GPI domain. Mesothelin peptide-specific T-cells response was then defined after harvest the supernatant by IFNγ production, quantified by ELISA (Mabtech, Stockholm, Sweden). The absolute values of IFN-γ production (in pg/ml) were plotted against the normalized average percentage of recognition. The normalization for average IFN-γ production was calculated as follows: ((total IFN-γ production (peptides 1 to 42)) / (IFN-γ production per peptide)) x 100. Immune response 'hotspots' were detected among MPF as well as GPI (**Fig. 5**) peptides, although more epitopes which induced higher average IFN-γ production among the patients were found in the GPI domain. This observations suggested that the GPI domain of mesothelin contains peptides that are potentially strong T-cell targets in human brain cancer.

### Example 6: Preparation, isolation, cultivation and intracellular cytokine staining of PBMC

To analyze the cytokine levels within Peripheral blood mononuclear cells (PBMCs) PBMCs were isolated from heparinized blood and subsequently cultivated. Plasma was removed following centrifugation of blood samples and stored at -20°C. PBMCs were isolated over a ficoll hypaque gradient as described previously [34] and cyropreserved in liquid nitrogen with freezing medium (fetal bovine serum (FBS) containing 10% DMSO). PBMCs were later thawed and cultured in T-cell medium (Cellgro medium supplemented with 5% pooled human AB serum and a cocktail of human cytokines (IL-2 (1000IU/ml), IL-15 (10ng/ml) and IL-21 (10ng/ml)) (Prospec, Ness-Ziona, Israel)). The culture was performed in a 6-well tissue culture plate (BD Falcon) at a density of 2 x $10^6$ cells/mL for 7 days. On day 7, the cells were stimulated with irradiated autologous PBMCs at a ratio of 1:10 and pulsed with 1 μg/ml of the peptide pool comprising full-length mesothelin (Q13421, non-overlapping - hereafter referred to as mesothelin peptide pool) in an adequate volume of T-cell medium. Three days later (day 10 of culture), 30 ng/mL of OKT3 antibody (anti-CD3 monoclonal antibody, Biolegend, CA, USA) was added to the culture and on day 18, cells were harvested for immunoassays. Per necessity, the culture medium was changed by replacing half the volume.

For intracellular cytokine staining 1.0 x $10^6$ PBMCs were stimulated with 1 μg/ml mesothelin peptide pool (Peptides & Elephants, Potsdam, Germany), medium control or PMA/Ionomycin (positive control, Sigma-Aldrich, St Louis, MI; USA) in R10 medium in the presence of brefeldin A (10μg/mL, Sigma-Aldrich St Louis, MI, USA) for 6 hours at 37°C. Stimulation was stopped by transferring the cells to a 4°C refrigerator, followed by washing with FACS buffer (PBS+0.1%FBS and staining with the following reagents: anti-CD3 Pacific blue (BD Biosciences, CA, USA), anti-CD4 PerCP-Cy5.5 (BD Biosciences, CA, USA) and anti-CD8 APC-Cy7 (BD Biosciences, CA, USA). After a 15-minute incubation at 4°C, the cells were washed and fixed with a Fix/Perm reagent (Beckman coulter, CA, USA), followed by a further 30-minute incubation at 4°C with an intracellular antibody mix (anti-TNFα APC (BD Biosciences CA, USA), anti-IFNγ PE-Cy7 (BD Biosciences CA, USA), anti-IL-2 PE (BD biosciences CA, USA) and anti-IL-17 FITC (Biolegend, CA, USA)). The stained cells were then washed with FACS buffer and acquired on a FACSAria flow cytometer (BD Biosciences, Stockholm, Sweden). Data was analyzed using FlowJo software (Treestar Inc.).

### Example 7: Mesothelin-specific response of GBM TIL

TIL generated from GBM patients were tested for their recognition of and ability to respond to mesothelin peptides. TIL from GBM tissue were isolated, cultured and expanded *in vitro* as previously described, using the IL-2/IL-15/IL-21 cytokine cocktail in T-cell medium as well as OKT3, in the presence of irradiated allogenic feeder cells ([35], [36]). Intracellular cytokine staining of TIL cultured with mesothelin peptide pool was performed exactly as described in Example 6.

Flow cytometric analysis of intracellular production of IFN-$\gamma$ by T cells was used as a measure of antigen-specific response following 6 hours of culture. CD4+ and CD8+ TIL produced a measurable antigen-specific response to mesothelin peptides, particularly IFN-$\gamma$ production by CD8+ TIL, while CD4+ TIL appeared to the major source of TNF-$\alpha$ (**Fig. 6**). Double negative T cells also produced an antigen-specific response to the peptides, although to a lesser extent than the former T-cell subsets (**Fig. 6**). Therefore, it is concluded that TIL generated from GBM tumors contain mesothelin-specific cells, which can respond to antigen with cytokine production.

**Example 8: Detection of mesothelin peptide and mesothelin-specific IgG in plasma of GBM patients**

Since humoral immune responses are clinically significant in cancer, the amount of mesothelin-specific antibodies in plasma obtained from patients with GBM (grade IV) as well as healthy individuals was quantified. For this purpose, plasma IgG antibodies specific for full-length mesothelin were determined by an indirect ELISA method developed in house. Briefly, 96-well U-bottom Maxisorp plates (Nunc, Roskilde, Denmark) were coated with either human IgG (Sigma, USA) as a reference standard ranging from 15000 ng/ml to 117 ng/ml in a seven-point serial dilution (1:2 ratio) or 1 $\mu$g/ml of full-length mesothelin antigen (R&D systems, Minneapolis, MN) in separate wells. The plate was incubated for one hour at 37°C and washed 3 times with wash buffer (PBS 0.05% + Tween 20), followed by blocking for 1 hours with PBS 2% + BSA 0.05% Tween 20 at room temperature (RT). After five washes, diluted patient plasma was added to the assay plate and incubated for a further two hours at RT. The plate was then washed five times, incubated with a secondary anti-human IgG monoclonal antibody (ALP conjugated, 1:1000 dilution, Mabtech, Stockholm, Sweden) for one hour at RT and washed five times thereafter. Para-nitrophenylphosphate (pNPP, Thermo Fisher Scientific, MA, USA) was added to the assay plate, followed by 45-minute incubation at RT in the dark. The reaction was stopped by adding 1N NaOH, and the optical density was measured at 405nm using a Vmax kinetic microplate reader (Molecular Devices). It was found that GBM patients had significantly higher titers of mesothelin-specific circulating IgG than healthy individuals (**Fig. 7A**).

In addition to the quantification of mesothelin-specific IgG in plasma, also the amount of mesothelin peptide in blood was measured. Quantitative determination of human mesothelin concentration in plasma was performed using the human mesothelin immunoassay kit (R&D systems, Minneapolis, MN) according to the manufacturer's protocol. The range of the standard curve was between 10 and 0.156 ng/mL, as previously reported ([37]). The amount of soluble full-length mesothelin in plasma of GBM patients was found to be in a median concentration of approximately 15 ng/ml (**Fig. 7B**). Thus, IgG molecules recognizing full-length mesothelin as well as the antigen itself are detectable in peripheral blood and have implication for immunodiagnosis of GBM.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

**REFERENCES**

1. WHO. World Cancer Report 2014. Lyon: International Agency for Research on Cancer, World Health Organisation; 2014.

2. Maher EA, Furnari FB, Bachoo RM, et al. Malignant glioma: genetics and biology of a grave matter. Genes & development 2001;15:1311-33.

3. Sowers JL, Johnson KM, Conrad C, Patterson JT, Sowers LC. The role of inflammation in brain cancer. Advances in experimental medicine and biology 2014;816:75-105.

4. Wen PY and Reardon DA. Neuro-oncology in 2015: Progress in glioma diagnosis, classification and treatment. Nature reviews Neurology. 2016; 12(2):69-70.

5. Vaios EJ, Nahed BV, Muzikansky A, Fathi AT, Dietrich J. Bone marrow response as a potential biomarker of outcomes in glioblastoma patients. Journal of neurosurgery 2016:1-7.

6. Larson EW, Peterson HE, Lamoreaux WT, MacKay AR, Fairbanks RK, Call JA, Carlson JD, Ling BC, Demakas JJ, Cooke BS and Lee CM. Clinical outcomes following salvage Gamma Knife radiosurgery for recurrent glioblastoma.

World journal of clinical oncology. 2014; 5(2):142-148.

7. Suryadevara CM, Verla T, Sanchez-Perez L, Reap EA, Choi BD, Fecci PE and Sampson JH. Immunotherapy for malignant glioma. Surgical neurology international. 2015; 6(Suppl 1):S68-77.

8. Pastan I and Hassan R. Discovery of mesothelin and exploiting it as a target for immunotherapy. Cancer research. 2014; 74(11):2907-2912.

9. Johnson MD, Vito F and O'Connell MJ. Mesothelin expression in the leptomeninges and meningiomas. The journal of histochemistry and cytochemistry : official journal of the Histochemistry Society. 2008; 56(6):579-585.

10. Hassan R and Ho M. Mesothelin targeted cancer immunotherapy. European journal of cancer. 2008; 44(1):46-53.

11. Iwahori K, Osaki T, Serada S, Fujimoto M, Suzuki H, Kishi Y, Yokoyama A, Hamada H, Fujii Y, Yamaguchi K, Hirashima T, Matsui K, Tachibana I, Nakamura Y, Kawase I and Naka T. Megakaryocyte potentiating factor as a tumor marker of malignant pleural mesothelioma: evaluation in comparison with mesothelin. Lung cancer. 2008; 62(1):45-54.

12. Bafna S, Kaur S and Batra SK. Membrane-bound mucins: the mechanistic basis for alterations in the growth and survival of cancer cells. Oncogene. 2010; 29(20):2893-2904.

13. Tozbikian G, Brogi E, Kadota K, Catalano J, Akram M, Patil S, Ho AY, Reis-Filho JS, Weigelt B, Norton L, Adusumilli PS and Wen HY. Mesothelin expression in triple negative breast carcinomas correlates significantly with basal-like phenotype, distant metastases and decreased survival. PloS one. 2014; 9(12):e114900.

14. Illei PB, Alewine C, Zahurak M, Cowan ML, Montgomery E, Hassan R, Xiang L, Pastan I and Kelly RJ. Mesothelin Expression in Advanced Gastroesophageal Cancer Represents a Novel Target for Immunotherapy. Applied immunohistochemistry & molecular morphology : AIMM / official publication of the Society for Applied Immunohistochemistry. 2016; 24(4):246-252.

15. Victor P. Eroschenko (2008). Di Fiore's atlas of histology with functional correlations

16. Nduom EK-E, Hadjipanayis CG, Van Meir EG. Glioblastoma Cancer Stem-like Cells - Implications for Pathogenesis and Treatment. Cancer Journal (Sudbury, Mass). 2012;18(1):100-106.

17. Armstrong DK, Laheru D, Ma WW, et al. A phase 1 study of MORAb-009, a monoclonal antibody against mesothelin in pancreatic cancer, mesothelioma and ovarian adenocarcinoma. J Clin Oncol. 2007;25 (Suppl):14041.

18. Chowdhury PS, Viner JL, Beers R, Pastan I. Isolation of a high-affinity stable single-chain Fv specific for mesothelin from DNA-immunized mice by phage display and construction of a recombinant immunotoxin with anti-tumor activity.

19. Chowdhury PS, Pastan I. Improving antibody affinity by mimicking somatic hypermutation in vitro. Nat Biotechnol. 1999;17:568-572

20. Le DT, Brockstedt DG, Nir-Paz R, Hampl J, Mathur S, Nemunaitis J, et al. A live-attenuated Listeria vaccine (ANZ-100) and a live-attenuated Listeria vaccine expressing mesothelin (CRS-207) for advanced cancers: phase I studies of safety and immune induction. Clin Cancer Res. 2012;18:858-68.

21. Ward et al., (1989) Nature 341:544-546)

22. Bird et al. (1988) Science 242:423-426;

23. Huston et al. (1988) Proc. Natl. Acad. Sci. USA 85:5879-5883;

24. Osbourn et al. (1998) Nat. Biotechnol. 16:778

25. Holliger, P. et al. (1993) Proc. Natl. Acad. Sci. USA 90:6444-6448;

26. Poljak, R. J. et al. (1994) Structure 2:1121-1123.

27. Wen and Reardon, Neuro-oncology, (2015)

28. Lagrelius et al., Cytokine. 2006;

29. Alvarez-Corrales et al., BMC infectious diseases. 2013

30. Gaines et al., Journal of immunological methods. 1996

31. Choi Y, Chang J. Viral vectors for vaccine applications. Clinical and Experimental Vaccine Research. 2013;2(2):97-105.

32. Maher et al., Genes & development. 2001;

33. Louis et al., Acta neuropathologica. 2016

34. Magalhaes et al., Immunology. 2010

35. Liu et al., 29th Annual Scientific Meeting of the Society for Immunotherapy of Cancer (SITC); 2015;

36. Meng et al., Journal of immunotherapy, 2016

37. Chen et al., PloS one, 2014

38. Dotti, G., Gottschalk, S., Savoldo, B., & Brenner, M. K. (2014). Design and Development of Therapies using Chimeric Antigen Receptor-Expressing T cells. Immunological Reviews, 257(1), 10.1111/imr.12131. http://doi.org/10.1111/imr.12131

## Claims

1. A mesothelin binding protein for use in the treatment of brain cancer in a patient, wherein said mesothelin binding protein comprises an epitope binding domain which specifically binds to an epitope containing amino acid sequence that is similar or identical to a section of the amino acid sequence of mammalian mesothelin precursor, in particular human mesothelin precursor (SEQ ID NO: 1).

2. The mesothelin binding protein for use according to claim 1, wherein the brain cancer is glioblastoma multiforme.

3. The mesothelin binding protein for use according to claim 1 or 2, wherein the section of the amino acid sequence of the human mesothelin precursor starts after amino acid 345 of SEQ ID NO: 1, preferably after amino acid 465 of SEQ ID NO: 1, more preferably said section has an amino acid sequence selected from SEQ ID NO: 2, SEQ ID NO: 3, SEQ ID NO: 4, SEQ ID NO: 5, SEQ ID NO: 6.

4. The mesothelin binding protein for use according to claim any of claims 1 to 3, wherein said mesothelin binding protein is an antibody or fragment thereof or a fusion protein of an antibody or fragment thereof.

5. The mesothelin binding protein for use according to any of claims 1 to 4, wherein said mesothelin binding protein is conjugated to an anti-cancer drug.

6. The mesothelin binding protein for use according to claim 5, wherein the anti-cancer drug is selected from the group consisting of a pseudomonas toxin, a cytokine, a cytokine such as IL-12, or a radioactive substance, and cisplatin.

7. The mesothelin binding protein for use according to any of claims 1 to 3, wherein said mesothelin binding protein is a chimeric antigen receptor (CAR), wherein preferably CAR is part of a CAR T-cell.

8. The mesothelin binding protein for use according to any of claims 1 to 3, wherein said mesothelin binding protein is a T-cell receptor (TCR).

9. An expanded T-cell for use in the treatment of brain cancer in a patient, wherein said T-cell comprises a TCR according to claim 11 and wherein said expanded T-cell is manufactured by clonal expansion of T-cells, preferably in the presence of a cytokine cocktail comprising at least two of interleukin 2 (IL-2), interleukin 15 (IL-15) and interleukin 21 (IL-21).

10. The expanded T-cell for use according to claim 9, wherein the T-cell is manufactured by clonal expansion of autologous T-cells of the patient, wherein the T-cells are preferably isolated from a body sample selected from the tumor, blood, tears, saliva, synovial fluid, placenta tissue, bone marrow, liquor and ascites.

11. The expanded T-cell for use according to claim 10, wherein the cultivation of expansion of the T-cell comprise at least one step of in which the T-cells are contacted with mesothelin peptides that have an amino acid sequence identical or similar to a section of the amino acid sequence of SEQ ID NO: 1, wherein preferably the section starts after amino acid 345 of SEQ ID NO: 1, preferably after amino acid 465 of SEQ ID NO: 1, more preferably the section has an amino acid sequence selected from SEQ ID NO: 2, SEQ ID NO: 3, SEQ ID NO: 4, SEQ ID NO: 5, SEQ ID NO: 6.

12. A cancer vaccine in the treatment or prevention of a brain cancer in a patient, wherein the cancer vaccine is selected from a vaccine peptide comprising an amino acid sequence that is similar or identical to the amino acid sequence of the human mesothelin precursor or a fragment thereof, or a nucleotide encoding said vaccine peptide, wherein preferably said vaccine protein comprises a fragment with a sequence similar or identical to SEQ ID NO: 2, SEQ ID NO: 3, SEQ ID NO: 4, SEQ ID NO: 5, SEQ ID NO: 6.

13. A method for diagnosing a patient suspected of a brain tumor, comprising the steps of

> a. Providing an unstimulated blood sample from the patient;
> b. Culturing the cells in the blood sample in the presence of a cytokine cocktail comprising at least two cytokines selected from comprising at least two of interleukin 2 (IL-2), interleukin 15 (IL-15) and interleukin 21 (IL-21) and a mesothelin peptide comprising an amino acid sequence that is similar or identical to the amino acid sequence of the human mesothelin precursor or a fragment thereof
> c. Measuring the IFNγ concentration in the culture;
> d. Comparing the concentration of IFNγ to an IFNγ threshold;

wherein a concentration of IFNγ above the IFNγ threshold is indicative for a Grade III or IV glioblastoma.

14. The method for diagnosing according to claim 13, further comprising the steps of:

> a. Measuring the concentration of mesothelin in the blood sample;
> b. Comparing the concentration of mesothelin to a mesothelin threshold.

and wherein a concentration of mesothelin above the mesothelin threshold IFNγ above the IFNγ threshold is indicative for a Grade IV glioblastoma.

15. The method for diagnosing according to claim 13 or 14, wherein the mesothelin threshold is 10 pg/mL blood.

**PaTu-1**      **glioma tissue**

Anti-Mesothelin Ab
+ Secondary Ab

Negative control
no Anti-Mesothelin
Ab + Secondary Ab

● Nucleus
● Mesothelin

**Fig. 1**

**Fig. 2**

Fig. 3

Fig. 4

Fig. 5

Fig. 6

Fig. 7

Fig. 8

**Fig. 9**

Fig. 10

Fig. 11

Fig. 12

Europäisches
Patentamt

European
Patent Office

Office européen
des brevets

# EUROPEAN SEARCH REPORT

Application Number

EP 17 16 6362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/110702 A1 (WU T C [US] ET AL) 30 April 2009 (2009-04-30) | 12 | INV. A61K39/00 |
| Y | * page 1, paragraph 0007 - paragraph 0008 * | 1,3-6 | A61K39/395 G01N33/50 G01N33/68 |
| | * page 2, paragraph 0014 * | | |
| | * page 6, paragraph 0049 * | | |
| | * page 11, paragraph 0086 - paragraph 0088 * | | |
| | ----- | | |
| X | JOHNSON MAHLON D ET AL: "Mesothelin expression in the leptomeninges and meningiomas", JOURNAL OF HISTOCHEMISTRY & CYTOCHEMISTRY, vol. 56, no. 6, June 2008 (2008-06), pages 579-585, XP002773174, ISSN: 0022-1554 | 1,4-6,12 | |
| Y | * abstract * * page 580, right-hand column, last paragraph - page 582, right-hand column, paragraph 1 * * page 583, right-hand column, paragraph 1 - page 584, right-hand column, paragraph 1 * | 1-8 | TECHNICAL FIELDS SEARCHED (IPC) A61K G01N |
| | ----- | | |
| X | QINGDA MENG ET AL.: "Enhanced tumor-infiltrating lymphocytes (eTIL) for cellular therapy of patients with pancreatic cancer or glioblastoma", JOURNAL OF IMMUNOTHERAPY OF CANCER, vol. 3, no. Supp2, 2015, page P35, XP002773175, | 9-11, 13-15 | |
| Y | * the whole document * | 2 | |
| | ----- | | |
| Y | WO 2015/188141 A2 (SLOAN KETTERING INST CANCER [US]; US HEALTH [US]) 10 December 2015 (2015-12-10) * page 82, line 15 - line 26 * | 7,8 | |
| | ----- | | |
| | -/-- | | |

The present search report has been drawn up for all claims

| Place of search | Date of completion of the search | Examiner |
|---|---|---|
| The Hague | 24 August 2017 | Montero Lopez, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

## EUROPEAN SEARCH REPORT

Application Number

EP 17 16 6362

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JOHNSON MAHLON D ET AL: "MUC16 Expression and Risk of Adenocarcinoma Metastases to Peritoneum, Pleura, Leptomeninges, and Brain", APPLIED IMMUNOHISTOCHEMISTRY & MOLECULAR MORPHOLOGY, vol. 18, no. 3, May 2010 (2010-05), pages 250-253, XP008185346, * page 250, right-hand column, paragraph 2 - paragraph 4 * * page 251, right-hand column, paragraph 3 - page 253, left-hand column, paragraph 1 * | 1,4-6 | |
| A | WO 2016/123122 A1 (BAYLOR COLLEGE MEDICINE [US]) 4 August 2016 (2016-08-04) * page 1, paragraph 0005 * * page 3, paragraph 0012 - page 4, paragraph 0013 * * page 5, paragraph 0018 * * page 9, paragraph 0044 - paragraph 0045 * * page 10, paragraph 0051 - page 11, paragraph 0052; claims 1, 10, 19, 20; examples * | 1-15 | |
| A | US 2014/004121 A1 (FANSLOW III WILLIAM CHRISTIAN [US] ET AL) 2 January 2014 (2014-01-02) * page 1, paragraph 0010 * * page 35, paragraph 00214 - column 36, paragraph 00215 * | 1-15 | TECHNICAL FIELDS SEARCHED (IPC) |
| T | WO 2015/090230 A1 (NOVARTIS AG [CH]; UNIV PENNSYLVANIA [US]; BEATTY GREGORY [US]; ENGELS) 25 June 2015 (2015-06-25) * page 219, paragraph 00709 - paragraph 00710; figure 16B * | 1-8 | |

The present search report has been drawn up for all claims

| Place of search | Date of completion of the search | Examiner |
|---|---|---|
| The Hague | 24 August 2017 | Montero Lopez, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 16 6362

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2017

| Patent document cited in search report | | Publication date | Patent family member(s) | | Publication date |
|---|---|---|---|---|---|
| US 2009110702 | A1 | 30-04-2009 | NONE | | |
| WO 2015188141 | A2 | 10-12-2015 | AU | 2015269219 A1 | 08-12-2016 |
| | | | CA | 2950763 A1 | 10-12-2015 |
| | | | EP | 3151854 A2 | 12-04-2017 |
| | | | JP | 2017518053 A | 06-07-2017 |
| | | | US | 2017081405 A1 | 23-03-2017 |
| | | | WO | 2015188141 A2 | 10-12-2015 |
| WO 2016123122 | A1 | 04-08-2016 | NONE | | |
| US 2014004121 | A1 | 02-01-2014 | AR | 091605 A1 | 18-02-2015 |
| | | | TW | 201420600 A | 01-06-2014 |
| | | | US | 2014004121 A1 | 02-01-2014 |
| | | | US | 2016340440 A1 | 24-11-2016 |
| | | | UY | 34885 A | 31-12-2013 |
| | | | WO | 2014004549 A2 | 03-01-2014 |
| WO 2015090230 | A1 | 25-06-2015 | AU | 2014366047 A1 | 16-06-2016 |
| | | | CA | 2931684 A1 | 25-06-2015 |
| | | | CN | 106459989 A | 22-02-2017 |
| | | | EP | 3083964 A1 | 26-10-2016 |
| | | | JP | 2017500869 A | 12-01-2017 |
| | | | SG | 11201604815R A | 28-07-2016 |
| | | | TW | 201529850 A | 01-08-2015 |
| | | | US | 2016311917 A1 | 27-10-2016 |
| | | | WO | 2015090230 A1 | 25-06-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150031624 A
- WO 2015189357 A1

### Non-patent literature cited in the description

- **NEEDLEMAN ; WUNSCH.** *J. Mol. Biol.,* 1970, vol. 48, 443-453
- **RICE.** EMBOSS: The European Molecular Biology Open Software Suite. *Trends Genet.,* 2000, vol. 16, 276-277
- **WHO.** World Cancer Report 2014. Lyon: International Agency for Research on Cancer. World Health Organisation, 2014
- **MAHER EA ; FURNARI FB ; BACHOO RM et al.** Malignant glioma: genetics and biology of a grave matter. *Genes & development,* 2001, vol. 15, 1311-33
- **SOWERS JL ; JOHNSON KM ; CONRAD C ; PATTERSON JT ; SOWERS LC.** The role of inflammation in brain cancer. *Advances in experimental medicine and biology,* 2014, vol. 816, 75-105
- **WEN PY ; REARDON DA.** Neuro-oncology in 2015: Progress in glioma diagnosis, classification and treatment. *Nature reviews Neurology,* 2016, vol. 12 (2), 69-70
- **VAIOS EJ ; NAHED BV ; MUZIKANSKY A ; FATHI AT ; DIETRICH J.** Bone marrow response as a potential biomarker of outcomes in glioblastoma patients. *Journal of neurosurgery,* 2016, 1-7
- **LARSON EW ; PETERSON HE ; LAMOREAUX WT ; MACKAY AR ; FAIRBANKS RK ; CALL JA ; CARLSON JD ; LING BC ; DEMAKAS JJ ; COOKE BS.** Clinical outcomes following salvage Gamma Knife radiosurgery for recurrent glioblastoma. *World journal of clinical oncology,* 2014, vol. 5 (2), 142-148
- **SURYADEVARA CM ; VERLA T ; SANCHEZ-PEREZ L ; REAP EA ; CHOI BD ; FECCI PE ; SAMPSON JH.** Immunotherapy for malignant glioma. *Surgical neurology international,* 2015, vol. 6 (1), 68-77
- **PASTAN I ; HASSAN R.** Discovery of mesothelin and exploiting it as a target for immunotherapy. *Cancer research,* 2014, vol. 74 (11), 2907-2912
- **JOHNSON MD ; VITO F ; O'CONNELL MJ.** Mesothelin expression in the leptomeninges and meningiomas. *The journal of histochemistry and cytochemistry : official journal of the Histochemistry Society,* 2008, vol. 56 (6), 579-585
- **HASSAN R ; HO M.** Mesothelin targeted cancer immunotherapy. *European journal of cancer,* 2008, vol. 44 (1), 46-53
- **IWAHORI K ; OSAKI T ; SERADA S ; FUJIMOTO M ; SUZUKI H ; KISHI Y ; YOKOYAMA A ; HAMADA H ; FUJII Y ; YAMAGUCHI K.** Megakaryocyte potentiating factor as a tumor marker of malignant pleural mesothelioma: evaluation in comparison with mesothelin. *Lung cancer,* 2008, vol. 62 (1), 45-54
- **BAFNA S ; KAUR S ; BATRA SK.** Membrane-bound mucins: the mechanistic basis for alterations in the growth and survival of cancer cells. *Oncogene,* 2010, vol. 29 (20), 2893-2904
- **TOZBIKIAN G ; BROGI E ; KADOTA K ; CATALANO J ; AKRAM M ; PATIL S ; HO AY ; REIS-FILHO JS ; WEIGELT B ; NORTON L.** Mesothelin expression in triple negative breast carcinomas correlates significantly with basal-like phenotype, distant metastases and decreased survival. *PloS one.,* 2014, vol. 9 (12), e114900
- **ILLEI PB ; ALEWINE C ; ZAHURAK M ; COWAN ML ; MONTGOMERY E ; HASSAN R ; XIANG L ; PASTAN I ; KELLY RJ.** Applied immunohistochemistry & molecular morphology : AIMM / official publication of the Society for Applied Immunohistochemistry. *Mesothelin Expression in Advanced Gastroesophageal Cancer Represents a Novel Target for Immunotherapy,* 2016, vol. 24 (4), 246-252
- **VICTOR P. EROSCHENKO.** Di Fiore's atlas of histology with functional correlations. 2008
- **NDUOM EK-E ; HADJIPANAYIS CG ; VAN MEIR EG.** Glioblastoma Cancer Stem-like Cells - Implications for Pathogenesis and Treatment. *Cancer Journal,* 2012, vol. 18 (1), 100-106
- **ARMSTRONG DK ; LAHERU D ; MA WW et al.** A phase 1 study of MORAb-009, a monoclonal antibody against mesothelin in pancreatic cancer, mesothelioma and ovarian adenocarcinoma. *J Clin Oncol.,* 2007, vol. 25, 14041

- **CHOWDHURY PS ; VINER JL ; BEERS R ; PASTAN I.** *Isolation of a high-affinity stable single-chain Fv specific for mesothelin from DNA-immunized mice by phage display and construction of a recombinant immunotoxin with anti-tumor activity*
- **CHOWDHURY PS ; PASTAN I.** Improving antibody affinity by mimicking somatic hypermutation in vitro. *Nat Biotechnol.,* 1999, vol. 17, 568-572
- **LE DT ; BROCKSTEDT DG ; NIR-PAZ R ; HAMPL J ; MATHUR S ; NEMUNAITIS J et al.** A live-attenuated Listeria vaccine (ANZ-100) and a live-attenuated Listeria vaccine expressing mesothelin (CRS-207) for advanced cancers: phase I studies of safety and immune induction. *Clin Cancer Res.,* 2012, vol. 18, 858-68
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **OSBOURN et al.** *Nat. Biotechnol.,* 1998, vol. 16, 778
- **HOLLIGER, P. et al.** *Proc. Natl. Acad. Sci. USA,* 1993, vol. 90, 6444-6448
- **POLJAK, R. J. et al.** *Structure,* 1994, vol. 2, 1121-1123
- **WEN ; REARDON.** *Neuro-oncology,* 2015
- **LAGRELIUS et al.** *Cytokine,* 2006
- **ALVAREZ-CORRALES et al.** *BMC infectious diseases,* 2013
- **GAINES et al.** *Journal of immunological methods,* 1996
- **CHOI Y ; CHANG J.** Viral vectors for vaccine applications. *Clinical and Experimental Vaccine Research,* 2013, vol. 2 (2), 97-105
- **MAHER et al.** *Genes & development,* 2001
- **LOUIS et al.** *Acta neuropathologica,* 2016
- **MAGALHAES et al.** *Immunology,* 2010
- **LIU et al.** *29th Annual Scientific Meeting of the Society for Immunotherapy of Cancer,* 2015
- **MENG et al.** *Journal of immunotherapy,* 2016
- **CHEN et al.** *PloS one,* 2014
- **DOTTI, G. ; GOTTSCHALK, S. ; SAVOLDO, B. ; BRENNER, M. K.** Design and Development of Therapies using Chimeric Antigen Receptor-Expressing T cells. *Immunological Reviews,* 2014, vol. 257 (1

SEQUENCE LISTING

<110>  polybiocept GmbH

<120>  Targeting Mesothelin in Brain Cancer

<130>  7027/P/1009-EP

<160>  6

<170>  PatentIn version 3.5

<210>  1
<211>  630
<212>  PRT
<213>  Homo sapiens

<400>  1

```
Met Ala Leu Pro Thr Ala Arg Pro Leu Leu Gly Ser Cys Gly Thr Pro
1               5                   10                  15


Ala Leu Gly Ser Leu Leu Phe Leu Leu Phe Ser Leu Gly Trp Val Gln
            20                  25                  30


Pro Ser Arg Thr Leu Ala Gly Glu Thr Gly Gln Glu Ala Ala Pro Leu
            35                  40                  45


Asp Gly Val Leu Ala Asn Pro Pro Asn Ile Ser Ser Leu Ser Pro Arg
        50                  55                  60


Gln Leu Leu Gly Phe Pro Cys Ala Glu Val Ser Gly Leu Ser Thr Glu
65                  70                  75                  80


Arg Val Arg Glu Leu Ala Val Ala Leu Ala Gln Lys Asn Val Lys Leu
                85                  90                  95


Ser Thr Glu Gln Leu Arg Cys Leu Ala His Arg Leu Ser Glu Pro Pro
            100                 105                 110


Glu Asp Leu Asp Ala Leu Pro Leu Asp Leu Leu Leu Phe Leu Asn Pro
            115                 120                 125


Asp Ala Phe Ser Gly Pro Gln Ala Cys Thr Arg Phe Phe Ser Arg Ile
        130                 135                 140


Thr Lys Ala Asn Val Asp Leu Leu Pro Arg Gly Ala Pro Glu Arg Gln
145                 150                 155                 160


Arg Leu Leu Pro Ala Ala Leu Ala Cys Trp Gly Val Arg Gly Ser Leu
                165                 170                 175
```

Leu Ser Glu Ala Asp Val Arg Ala Leu Gly Gly Leu Ala Cys Asp Leu
        180             185             190

Pro Gly Arg Phe Val Ala Glu Ser Ala Glu Val Leu Leu Pro Arg Leu
        195             200             205

Val Ser Cys Pro Gly Pro Leu Asp Gln Asp Gln Gln Glu Ala Ala Arg
    210             215             220

Ala Ala Leu Gln Gly Gly Gly Pro Pro Tyr Gly Pro Pro Ser Thr Trp
225             230             235             240

Ser Val Ser Thr Met Asp Ala Leu Arg Gly Leu Leu Pro Val Leu Gly
            245             250             255

Gln Pro Ile Ile Arg Ser Ile Pro Gln Gly Ile Val Ala Ala Trp Arg
        260             265             270

Gln Arg Ser Ser Arg Asp Pro Ser Trp Arg Gln Pro Glu Arg Thr Ile
        275             280             285

Leu Arg Pro Arg Phe Arg Arg Glu Val Glu Lys Thr Ala Cys Pro Ser
    290             295             300

Gly Lys Lys Ala Arg Glu Ile Asp Glu Ser Leu Ile Phe Tyr Lys Lys
305             310             315             320

Trp Glu Leu Glu Ala Cys Val Asp Ala Ala Leu Leu Ala Thr Gln Met
            325             330             335

Asp Arg Val Asn Ala Ile Pro Phe Thr Tyr Glu Gln Leu Asp Val Leu
        340             345             350

Lys His Lys Leu Asp Glu Leu Tyr Pro Gln Gly Tyr Pro Glu Ser Val
        355             360             365

Ile Gln His Leu Gly Tyr Leu Phe Leu Lys Met Ser Pro Glu Asp Ile
    370             375             380

Arg Lys Trp Asn Val Thr Ser Leu Glu Thr Leu Lys Ala Leu Leu Glu
385             390             395             400

Val Asn Lys Gly His Glu Met Ser Pro Gln Ala Pro Arg Arg Pro Leu
            405             410             415

Pro Gln Val Ala Thr Leu Ile Asp Arg Phe Val Lys Gly Arg Gly Gln
        420             425             430

```
Leu Asp Lys Asp Thr Leu Asp Thr Leu Thr Ala Phe Tyr Pro Gly Tyr
        435             440         445

Leu Cys Ser Leu Ser Pro Glu Glu Leu Ser Ser Val Pro Pro Ser Ser
        450             455         460

Ile Trp Ala Val Arg Pro Gln Asp Leu Asp Thr Cys Asp Pro Arg Gln
465             470             475             480

Leu Asp Val Leu Tyr Pro Lys Ala Arg Leu Ala Phe Gln Asn Met Asn
                485             490             495

Gly Ser Glu Tyr Phe Val Lys Ile Gln Ser Phe Leu Gly Gly Ala Pro
            500             505             510

Thr Glu Asp Leu Lys Ala Leu Ser Gln Gln Asn Val Ser Met Asp Leu
            515             520             525

Ala Thr Phe Met Lys Leu Arg Thr Asp Ala Val Leu Pro Leu Thr Val
        530             535             540

Ala Glu Val Gln Lys Leu Leu Gly Pro His Val Glu Gly Leu Lys Ala
545             550             555             560

Glu Glu Arg His Arg Pro Val Arg Asp Trp Ile Leu Arg Gln Arg Gln
            565             570             575

Asp Asp Leu Asp Thr Leu Gly Leu Gly Leu Gln Gly Gly Ile Pro Asn
            580             585             590

Gly Tyr Leu Val Leu Asp Leu Ser Met Gln Glu Ala Leu Ser Gly Thr
        595             600             605

Pro Cys Leu Leu Gly Pro Gly Pro Val Leu Thr Val Leu Ala Leu Leu
        610             615             620

Leu Ala Ser Thr Leu Ala
625             630


<210>  2
<211>  15
<212>  PRT
<213>  Homo sapiens

<400>  2

Tyr Glu Gln Leu Asp Val Leu Lys His Lys Leu Asp Glu Leu Tyr
1               5               10              15
```

20

```
<210>   3
<211>   15
<212>   PRT
<213>   Homo sapiens

<400>   3

Trp Ala Val Arg Pro Gln Asp Leu Asp Thr Cys Asp Pro Arg Gln
1               5                   10                  15


<210>   4
<211>   15
<212>   PRT
<213>   Homo sapiens

<400>   4

Leu Asp Val Leu Tyr Pro Lys Ala Arg Leu Ala Phe Gln Asn Met
1               5                   10                  15


<210>   5
<211>   15
<212>   PRT
<213>   Homo sapiens

<400>   5

Leu Gln Gly Gly Ile Pro Asn Gly Tyr Leu Val Leu Asp Leu Ser
1               5                   10                  15


<210>   6
<211>   14
<212>   PRT
<213>   Homo sapiens

<400>   6

Met Gln Glu Ala Leu Ser Gly Thr Pro Cys Leu Leu Gly Pro
1               5                   10
```